# EUROPEAN PATENT APPLICATION

(11) **EP 2 036 988 A1**
(43) Date of publication of application: **18.03.2009**
(21) Application number: 07122919.9
(22) Date of filing: 11.12.2007
(51) Int. Cl.: C12Q 1/68

(54) **A method for predicting the response of a tumor in a patient suffering from or at risk of developing recurrent gynecologic cancer towards a chemotherapeutic agent**

(30) Priority: 12.09.2007 DE 102007043455
(71) Applicant: Siemens Healthcare Diagnostics GmbH, 65760 Eschborn (DE)
(72) Inventor: Gehrmann, Mathias, 51373, Leverkusen (DE); Brase, Jan Christoph, 21255, Tostedt (DE)
(74) Representative: Maier, Daniel Oliver

(57) **Abstract**

The present invention relates to a method for predicting a response of a tumor in a patient suffering from or at risk of developing recurrent gynecologic cancer towards a chemotherapeutic agent, said method comprising the steps of: a) obtaining a biological sample from said patient; b) determining the pattern of expression level of at least one gene of the group comprising AKR1C1, MLPH, ESR1, PGR, COMP, DCN, IGKC, CCL5, FBN1 and/or UBE2C, or of genes coregulated therewith, in said sample; c) comparing the pattern of expression levels determined in (b) with one or several reference pattern(s) of expression levels; d) identifying at least one marker gene; e) determining a molecular subtype for said sample on the basis of (d); and f) predicting from said molecular subtype response of a tumor for a chemotherapeutic agent, wherein the molecular subtype is selected from the group comprising the subtypes basal, stromal-high, stromal-low, luminal A, immune system-high, immune system-low, proliferation-high and/or proliferation-low.

SEQUENCE LISTING

<110>  Siemens AG

<120>  A method for predicting the response of a tumor in a patient
       suffering from or at risk of developing recurrent gynecologic
       cancer towards a chemotherapeutic agent

<130>  200717108

<160>  30

<170>  PatentIn version 3.3

<210>  1
<211>  26
<212>  DNA
<213>  artificial

<220>
<223>  MLPH

<400>  1
ccaaatgcag acccttcaag tgaggc                                          26


<210>  2
<211>  20
<212>  DNA
<213>  artificial

<220>
<223>  MLPH

<400>  2
tcgagtggct gggaaacttg                                                 20


<210>  3
<211>  20
<212>  DNA
<213>  artificial

<220>
<223>  MLPH

<400>  3
agatagggca cagccattgc                                                 20


<210>  4
<211>  19
<212>  DNA
<213>  artificial

<220>
<223>  ESR1

<400>  4
atgccctttt gccgatgca                                                  19


<210>  5
<211>  24
<212>  DNA
<213>  artificial

<220>

<223>  ESR1

<400>  5
gccaaattgt gtttgatgga ttaa                                              24


<210>  6
<211>  27
<212>  DNA
<213>  artificial

<220>
<223>  ESR1

<400>  6
gacaaaaccg agtcacatca gtaatag                                           27


<210>  7
<211>  24
<212>  DNA
<213>  artificial

<220>
<223>  PGR

<400>  7
ttgatagaaa cgctgtgagc tcga                                              24


<210>  8
<211>  22
<212>  DNA
<213>  artificial

<220>
<223>  PGR

<400>  8
agctcatcaa ggcaattggt tt                                                22


<210>  9
<211>  28
<212>  DNA
<213>  artificial

<220>
<223>  PGR

<400>  9
acaagatcat gcaagttatc aagaagtt                                          28


<210>  10
<211>  27
<212>  DNA
<213>  artificial

<220>
<223>  COMP

<400>  10
caggagaaca tcatctgggc caacctg                                           27


<210>  11
<211>  20

<212>  DNA
<213>  artificial

<220>
<223>  COMP

<400>  11
gacagcaacg tggtcttgga                                                    20


<210>  12
<211>  21
<212>  DNA
<213>  artificial

<220>
<223>  COMP

<400>  12
atggtgtcat tgcagcggta a                                                  21


<210>  13
<211>  26
<212>  DNA
<213>  artificial

<220>
<223>  IGKC

<400>  13
agcagcctgc agcctgaaga ttttgc                                             26


<210>  14
<211>  24
<212>  DNA
<213>  artificial

<220>
<223>  IGKC

<400>  14
gatctgggac agaattcact ctca                                               24


<210>  15
<211>  19
<212>  DNA
<213>  artificial

<220>
<223>  IGKC

<400>  15
gccgaacgtc caagggtaa                                                     19


<210>  16
<211>  23
<212>  DNA
<213>  artificial

<220>
<223>  CCL5

<400>  16
ctcggacacc acaccctgct gct                                                23

<210>  17
<211>  19
<212>  DNA
<213>  artificial

<220>
<223>  CCL5

<400>  17
ctgcatctgc ctccccata                                                    19


<210>  18
<211>  18
<212>  DNA
<213>  artificial

<220>
<223>  CCL5

<400>  18
agtgggcggg caatgtag                                                     18


<210>  19
<211>  21
<212>  DNA
<213>  artificial

<220>
<223>  DCN

<400>  19
tcttttcagc aacccggtcc a                                                 21


<210>  20
<211>  22
<212>  DNA
<213>  artificial

<220>
<223>  DCN

<400>  20
aaggcttctt attcgggtgt ga                                                22


<210>  21
<211>  22
<212>  DNA
<213>  artificial

<220>
<223>  DCN

<400>  21
tggatggctg tatctcccag ta                                                22


<210>  22
<211>  26
<212>  DNA
<213>  artificial

<220>

<223>  FBN1

<400>  22
ctcagtggcc agaggatcac cagtgc                                          26


<210>  23
<211>  21
<212>  DNA
<213>  artificial

<220>
<223>  FBN1

<400>  23
gtctgggagg accaggaaac a                                               21


<210>  24
<211>  21
<212>  DNA
<213>  artificial

<220>
<223>  FBN1

<400>  24
tgcacatgct gtgatgaagg a                                               21


<210>  25
<211>  25
<212>  DNA
<213>  artificial

<220>
<223>  UBE2C

<400>  25
tgaacacaca tgctgccgag ctctg                                           25


<210>  26
<211>  27
<212>  DNA
<213>  artificial

<220>
<223>  UBE2C

<400>  26
cttctaggag aacccaacat tgatagt                                         27


<210>  27
<211>  27
<212>  DNA
<213>  artificial

<220>
<223>  UBE2C

<400>  27
gtttcttgca ggtacttctt aaaagct                                         27


<210>  28
<211>  28

<212>  DNA
<213>  artificial

<220>
<223>  AKR1C1

<400>  28
cctatgcgcc tgcagaggtt cctaaaag                                          28


<210>  29
<211>  20
<212>  DNA
<213>  artificial

<220>
<223>  AKR1C1

<400>  29
catgcctgtc ctgggatttg                                                   20


<210>  30
<211>  23
<212>  DNA
<213>  artificial

<220>
<223>  AKR1C1

<400>  30
aatttggtgg cctctaaagc ttt                                               23

## Description

### Field of the invention

The present invention relates to methods for predicting the response of a tumor in a patient suffering from or at risk of developing recurrent gynecologic cancer towards a chemotherapeutic agent.

### Background of the invention

Every fourth cancer finding in women is breast cancer. Therewith, breast cancer is the most common cancer and the second most common cause of death among women in western industrial nations (Jemal et al., 2007) ¹. It is estimated that every eighth to tenth woman will develop breast cancer during her lifetime. With a total share of 10 % it is the third most common cancer worldwide (Veronesi et al., 2005) ². With an incidence of 130 cases per 100000 women there are about 55000 new cases in Germany annually from which 18000 cases will cause death (GEKID, 2006) ³.

The mamma carcinoma is a very heterogonous disease with many subtypes. Therefore, even pathologically similar tumors show a different clinical development towards the same therapy. For this reason, the current histopathological markers can not predict the clinical response adequate. Therefore, it is very difficult to perform an optimized therapy. Hence, a therapy often will be chosen due to empirical experiences, and most of the women will be treated systemically as a precaution (Bast et al., 2001; Goldhirsch et al., 2005) ^{4,5}.

Usually, a combination of several agents will be used since due to the different therapeutic mechanisms of the single chemotherapeutic agents the development of crossresistances is unlikely. A very common combination therapy used already many years as standard therapy is CMF (Cyclophosphamid, Methotrexat and 5-Fluorouracil). These three chemotherapeutic agents have different molecular mechanisms of action. 5-Fluorouracil inhibits for instance the Thymidylate Synthetase irreversible and therewith the DNA synthesis (Longley et al., 2003) ⁶.

Over the years new chemotherapeutic agents were identified, which might have an advantage for the treatment of patients. In clinical practice there exists the problem that a new substance can not be tested independently from other agents since they are added to an already established combination therapy. With the results from this therapy it can be determined, whether there could arise an improvement over the standard therapy.

Thus, after the discovery of anthracyclines it could be demonstrated in clinical studies that for instance the combination therapy EC/AC (Epirubicin and Doxorubicin, respectively, and Cyclophosphamid) has an advantage over the CMF therapy. Anthracyclines are intercalators, which can incorporate into the DNA, dissolve their structure and inhibit the topoisomerase II (Capranico et al., 1989) ⁷. The administration of an anthracycline leads to a reduction of recurrent incidences about 12 % and to a reduction of the death rate about 11 % in comparison to a CMF therapy (Misset et al., 1996) ⁸.

In current clinical studies it could be demonstrated that the completion of an anthacycline based chemotherapy with a taxane leads to an additional advantage of survival (Nabholtz et al., 1999; Henderson et al., 2003; Bishop et al., 1999) ^{9,10,11}. The agent Paclitaxel was the first taxane, which was used for breast cancer therapy. Paclitaxel binds to the beta-tubuli-unities of the mikrotubuli and stabilizes them (Parness and Horwitz, 1981) ¹²_{.}

Newly, platin derivatives (Carboplatin, Cisplatin) are used for the treatment of the mamma carcinoma. The cytotoxic effect of the platin derivatives is caused by a cross-linking of DNA single strands and double strands, which are disabled thereby.

One problem of chemotherapy is development of resistances of some tumors towards single or several agents which can inhibit the success of a chemotherapy. To date, only a few resistance mechanisms are known. Furthermore, the order of the application seems to be important regarding crossresistances (Paridaens et al., 2000) ¹³. Thus, the identification at an early stage of those resistances is very important to change a therapy in good time. However, it would be optimal to evaluate possible resistances already before a treatment.

Another problem of chemotherapy is occurrence of adverse effects that might be life threatening or severely impairing the quality of life.

In the last years the heterogeneity and complexity of mamma carcinoma have been analyzed on molecular level to analyze which genes are expressed in these tumors.

An already known approach for the identification of predictive gene signatures is the cultivation of cell lines with the purpose to compare the gene expression of resistant and sensitive breast cancer tumor cells. Different genes could be identified which could be appropriate for the prediction of a tumor response towards single chemotherapeutic agents (Villeneuve et al., 2006; Gyorffy et al., 2006) ^{14,15}. Since the expression profile of cell lines in the in vitro situation is much different from the *in vivo* situation of primary tumor tissues (Ross and Perou, 2001) ¹⁶, it has to be evaluated, to which extent the identified genes could be appropriate for prediction in clinical practice.

In research neoadjuvant chemotherapy is very important as well since breast tumor response towards chemotherapeutic agents can be directly analyzed via the tumor reduction status.

A common approach is to isolate RNA from primary tumor tissues for gene expression analysis before neoadjuvant chemotherapy. The chemotherapeutic success can be directly evaluated via tumor reduction and correlated with the gene expression data. In several neoadjuvant studies predictive gene signatures could be identified (Ayers et al., 2004; Hess et al., 2006; Gianni et al., 2005; Thuerigen et al., 2006) ^{17,18,19,20}. In most of the studies neoadjuvant combination therapies instead of monotherapies have been analyzed. Thus, it is difficult to identify the cause of resistances as well as to transfer the identified gene signatures upon other combination therapies.

To date, there exists no reliable predictive marker which can predict the response towards a chemotherapeutic agent in breast cancer. To date, no gene signature could be validated in an independent dataset.

Therefore, a need exists to identify those patients who are likely to respond to a chemotherapeutic agent by providing predictive information. There is a great need for predictive methods that can assist the practicing physician to make treatment choices based on reliable analysis.

### Definitions

Unless defined otherwise, technical and scientific terms used herein have the same meaning as commonly understood by one of ordinary skill in the art to which this invention belongs.

The term "prediction", as used herein, relates to an individual assessment of the malignancy of a tumor, or to the expected survival rate (DFS, disease free survival) of a patient, if the tumor is treated with a given therapy.

"Prediction of the response to chemotherapy", within the meaning of the invention, shall be understood to be the act of determining a likely outcome of a chemotherapy in a patient inflicted with cancer. The prediction of a response is preferably made with reference to probability values for reaching a desired or non-desired outcome of the chemotherapy. The predictive methods of the present invention can be used clinically to make treatment decisions by choosing the most appropriate treatment modalities for any particular patient.

The "response of a tumor to chemotherapy", within the meaning of the invention, relates to any response of the tumor to chemotherapy, preferably to a change in tumor mass and/or volume after initiation of neoadjuvant chemotherapy. Tumor response may be assessed in a neoadjuvant situation where the size of a tumor after systemic intervention can be compared to the initial size and dimensions as measured by CT, PET, mammogram, ultrasound or palpation, usually recorded as "clinical response" of a patient. Response may also be assessed by caliper measurement or pathological examination of the tumor after biopsy or surgical resection. Response may be recorded in a quantitative fashion like percentage change in tumor volume or in a qualitative fashion like "no change" (NC), "partial remission" (PR), "complete remission" (CR) or other qualitative criteria. Assessment of tumor response may be done early after the onset of neoadjuvant therapy e.g. after a few hours, days, weeks or preferably after a few months. A typical endpoint for response assessment is upon termination of neoadjuvant chemotherapy or upon surgical removal of residual tumor cells and/or the tumor bed. This is typically three month after initiation of neoadjuvant therapy.

The term "response marker" relates to a marker which can be used to predict the clinical response of a patient towards a given treatment.

The terms "cancer" and "cancerous" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant conditions, neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer and primary carcinomas are included.

As used herein, the term "gynecologic cancers" refers to cancer which are diagnosed in female breast and reproductive organs that include the uterus, ovaries, cervix, fallopian tubes, vulva, and vagina. Examples of gynecologic cancers include, but are not limited to breast cancer, ovarian cancer, vulvar cancer, vaginal cancer, tubal cancer, endometrian cancer and/or cervical cancer. As used herein, the term "endometrian cancer", also called endometrial cancer or uterine cancer, includes malignant growth of cells in the endometrium, the lining of the uterus.

The term "tumor" as used herein, refers to all neoplastic cell growth and proliferation, whether malignant or benign, and all pre-cancerous and cancerous cells and tissues.

The term "determining the status" as used herein, refers to a measurable property of a gene and its products, especially on the nucleotide level and the gene level including mutation status and gene expression status. A number of parameters to determine the status of a gene and its products can be used including, but not limited to, determining the level of protein expression, the amplification or expression status on RNA level or DNA level, of polynucleotides and of polypeptides, and the analysis of haplotype or the mutation status of the gene. An exemplary determinable property correlated with the status of estrogen receptor or progesterone receptor is the amount of the estrogen receptor or progesterone receptor RNA, DNA or other polypeptide in the sample or the presence of nucleotide polymorphisms.

The term "biological sample", as used herein, refers to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, or cells there from. Biological samples may also include sections of tissues such as frozen or fixed sections taken for histological purposes or microdissected cells or extracellular parts thereof. A biological sample to be analyzed is tissue material from neoplastic lesion taken by aspiration or punctuation, excision or by any other surgical method leading to biopsy or resected cellular material. Such biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The terms "regulated" or "regulation" as used herein refer to both upregulation [i.e., activation or stimulation (e.g., by agonizing or potentiating] and down regulation [i.e., inhibition or suppression (e.g., by antagonizing, decreasing or inhibiting)].

The term "transcriptome" relates to the set of all messenger RNA (mRNA) molecules, or "transcripts", produced in one or a population of cells. The term can be applied to the total set of transcripts in a given organism, or to the specific subset of transcripts present in a particular cell type. Unlike the genome, which is roughly fixed for a given cell line (excluding mutations), the transcriptome can vary with external environmental conditions. Because it includes all *mRNA* transcripts in the cell, the transcriptome reflects the genes that are being actively expressed at any given time, with the exception of mRNA degradation phenomena such as transcriptional attenuation. The discipline of transcriptom-ics examines the expression level of mRNAs in a given cell population, often using high-throughput techniques based on DNA microarray technology.

The term "expression levels" refers, e.g., to a determined level of gene expression. The term "pattern of expression levels" refers to a determined level of gene expression compared either to a reference gene (e.g. housekeeper or inversely regulated genes) or to a computed average expression value (e.g. in DNA-chip analyses). A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells of blood vessels as well as cells of the immune system (e.g. lymphocytes, macrophages, killer cells).

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

"Primer pairs" and "probes", within the meaning of the invention, shall have the ordinary meaning of this term which is well known to the person skilled in the art of molecular biology. In a preferred embodiment of the invention "primer pairs" and "probes" shall be understood as being polynucleotide molecules having a sequence identical, complementary, homologous, or homologous to the complement of regions of a target polynucleotide which is to be detected or quantified. In yet another embodiment, nucleotide analogues are also comprised for usage as primers and/or probes.

The term "marker" refers to a biological molecule, e.g., a nucleic acid, peptide, protein, hormone, etc., whose presence or concentration can be detected and correlated with a known condition, such as a disease state.

The term "marker gene," as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in particular. A marker gene may also have the characteristics of a target gene.

The term "expression level", as used herein, relates to the process by which a gene's DNA sequence is converted into functional protein (i.e. ligands) and particularly to the amount of said conversion.

When used in reference to a single-stranded nucleic acid sequence, the term "substantially homologous" refers to any probe that can hybridize (i.e., it is the complement of) the single-stranded nucleic acid sequence under conditions of low stringency as described above.

As used herein, the term "hybridization" is used in reference to the pairing of complementary nucleic acids.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR)

The term "determining the protein level", as used herein, refers to methods which allow the quantitative and/or qualitative determination of one or more proteins in a sample. These methods include, among others, protein purification, including ultracentrifugation, precipitation and chromatography, as well as protein analysis and determination, including the use protein microarrays, two-hybrid screening, blotting methods including western blot, one- and two dimensional gelelectrophoresis, isoelectric focusing and the like.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

### Object of the invention

It is one object of the present invention to provide an improved method for the prediction of a response of a tumor in a patient suffering from gynecologic cancer towards a chemotherapeutic agent to current status tests.

The present invention provides new diagnostic criteria for the treatment of gynecologic cancer and an optimal predictive gene signature for different chemotherapeutic agents.

These objects are met with methods and means according to the independent claims of the present invention. The dependent claims are related to preferred embodiments. It is yet to be understood that value ranges delimited by numerical values are to be understood to include the said delimiting values.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a response of a tumor in a patient suffering from or at risk of developing recurrent gynecologic cancer, preferably breast cancer, towards a chemotherapeutic agent. Said method comprises the steps of:
a) obtaining a biological sample from said patient;
b) determining the pattern of expression level of at least one gene of the group comprising AKR1C1, MLPH, ESR1, PGR, COMP, DCN, IGKC, CCL5, FBN1 and/or UBE2C, or of genes coregulated therewith, in said sample;
c) comparing the pattern of expression levels determined in (b) with one or several reference pattern(s) of expression levels;
d) identifying at least one marker gene;
e) determining a molecular subtype for said sample on the basis of (d); and
f)predicting from said molecular subtype response of a tumor for a chemotherapeutic agent,
   wherein the molecular subtype is selected from the group comprising the subtypes basal, stromal-high, stromal-low, luminal A, immune system-high, immune system-low, proliferation-high and/or proliferation-low.

The molecular subtypes are divided into said groups based on the gene expression of the tumor.

It is vital to the present invention that, in addition to the genes enumerated under item b), one or more genes coregulated with at least one of these genes may be used in addition, or in replacement, of the said genes. Replacement of the said genes with one or more genes coregulated therewith is advantageous in cases, where determining the expression level of the genes enumerated under item b) is critical. Further, an additional use of coregulated genes increases the specificity of said method.

Such genes may be selected from the following table.

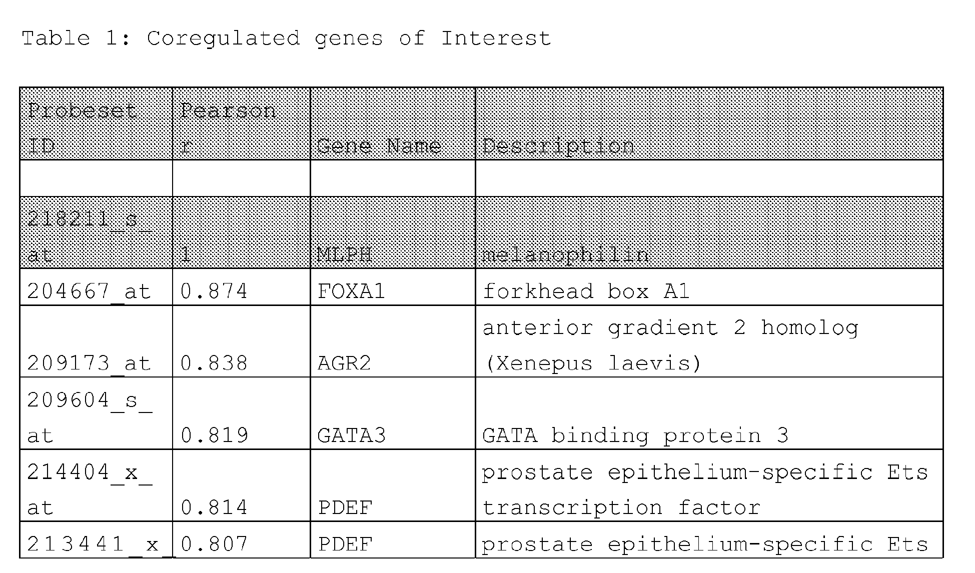

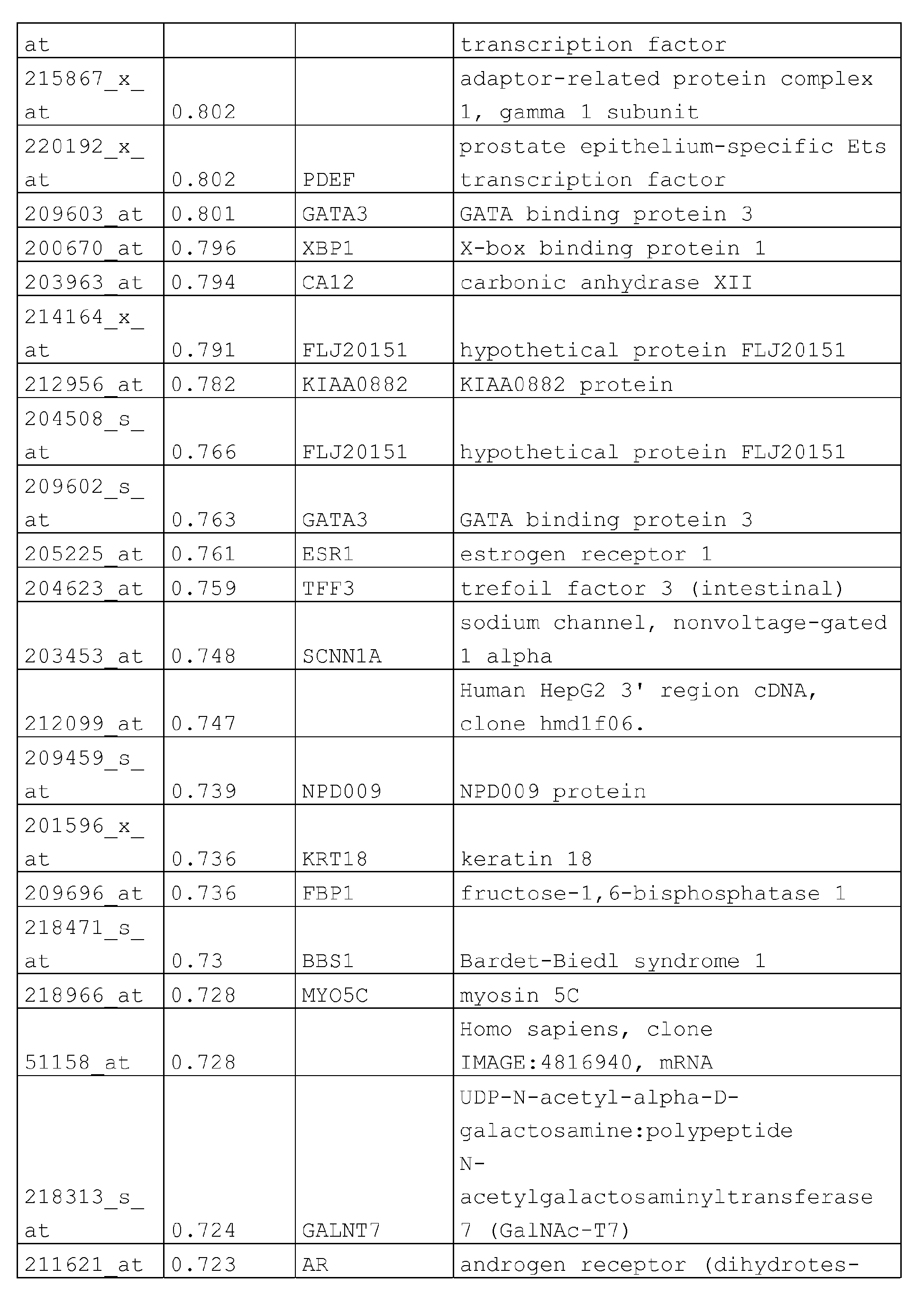

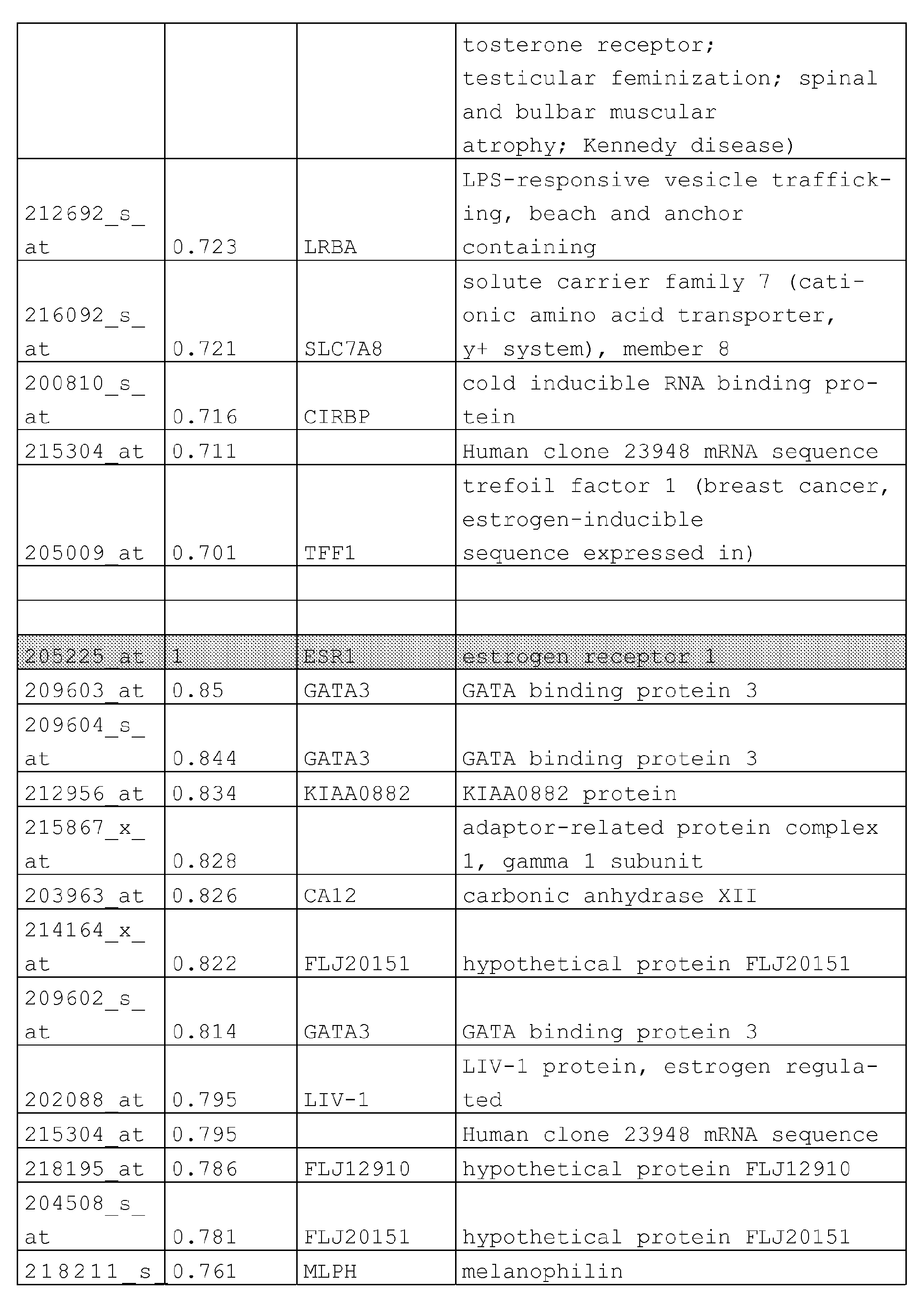

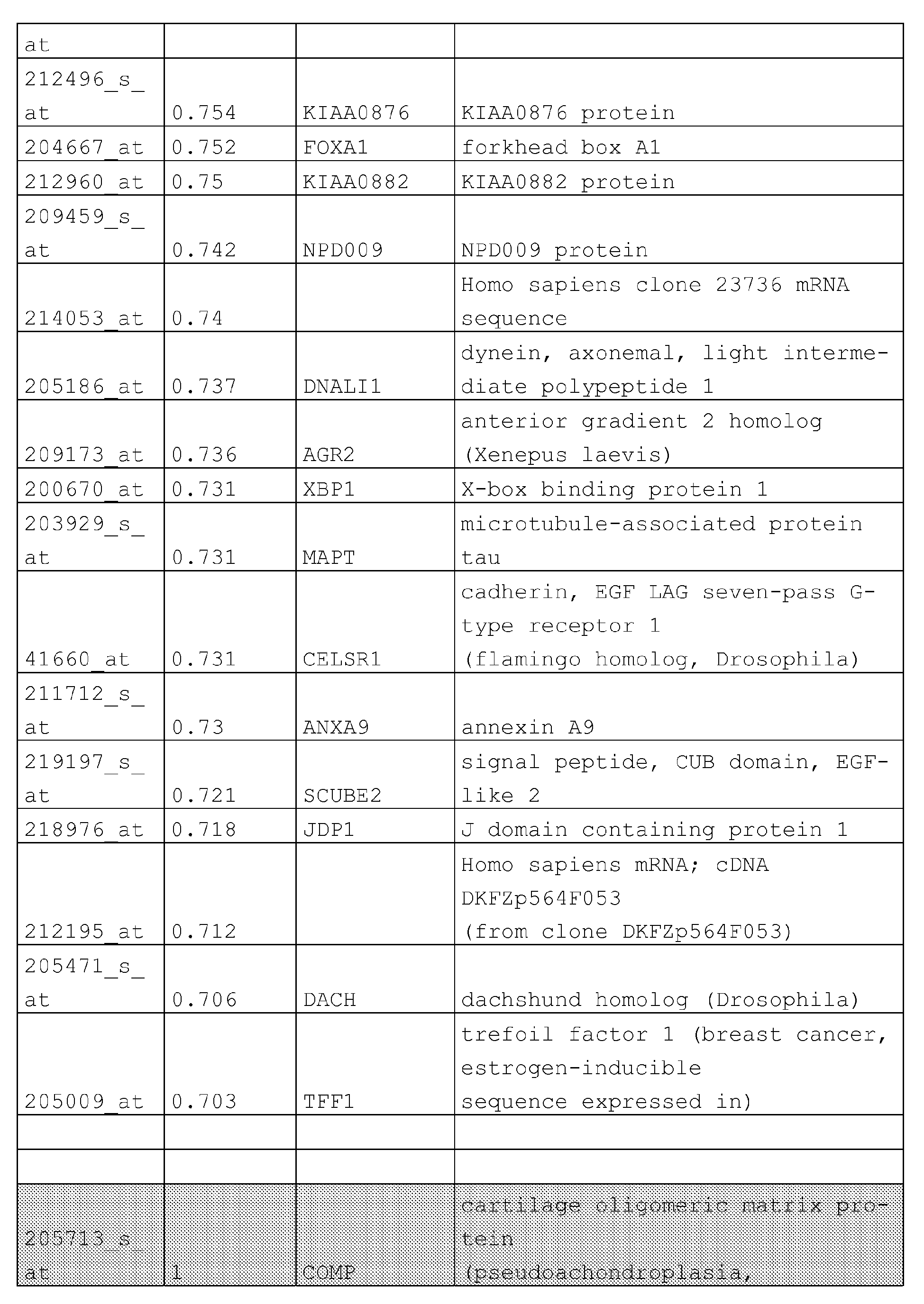

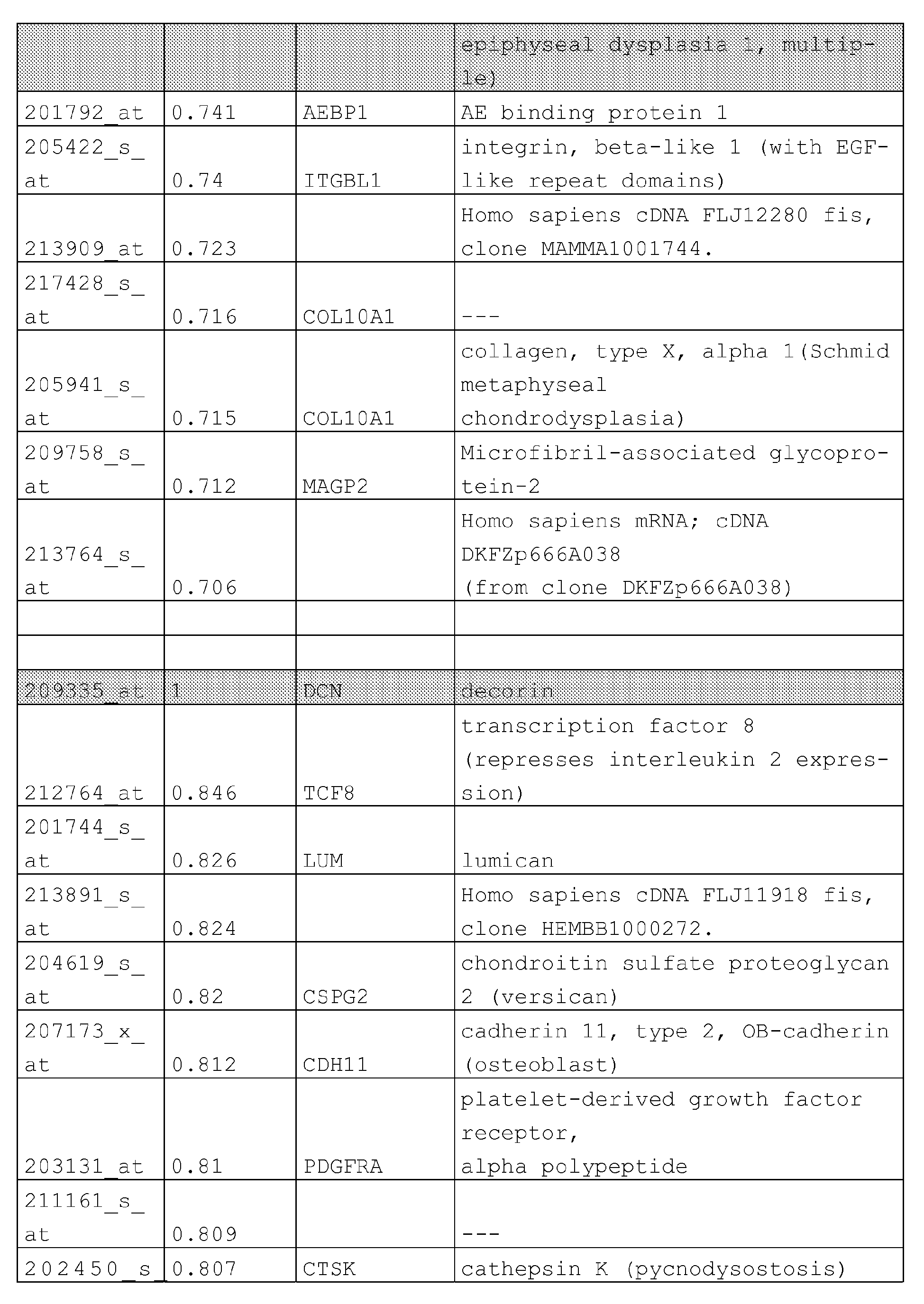

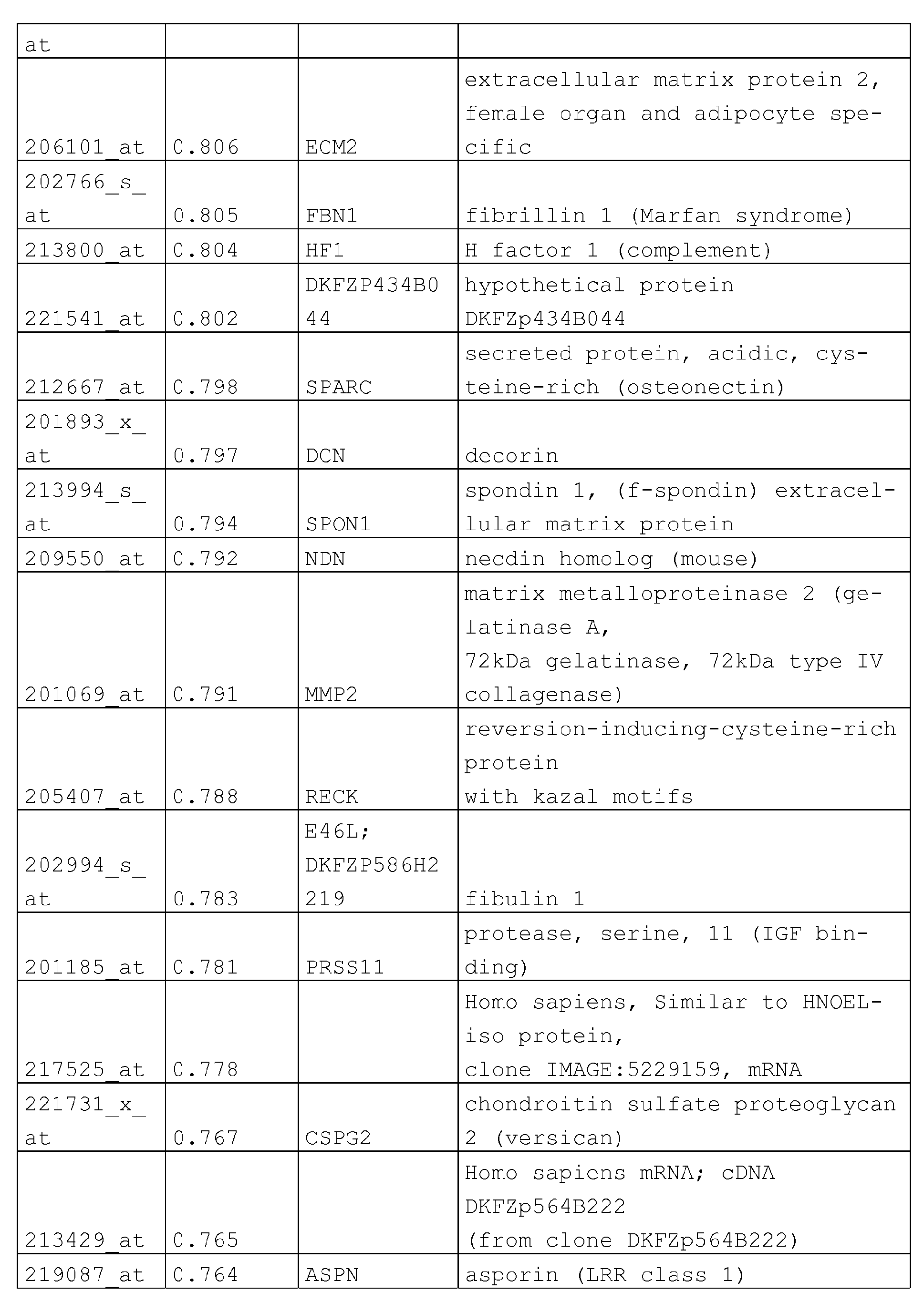

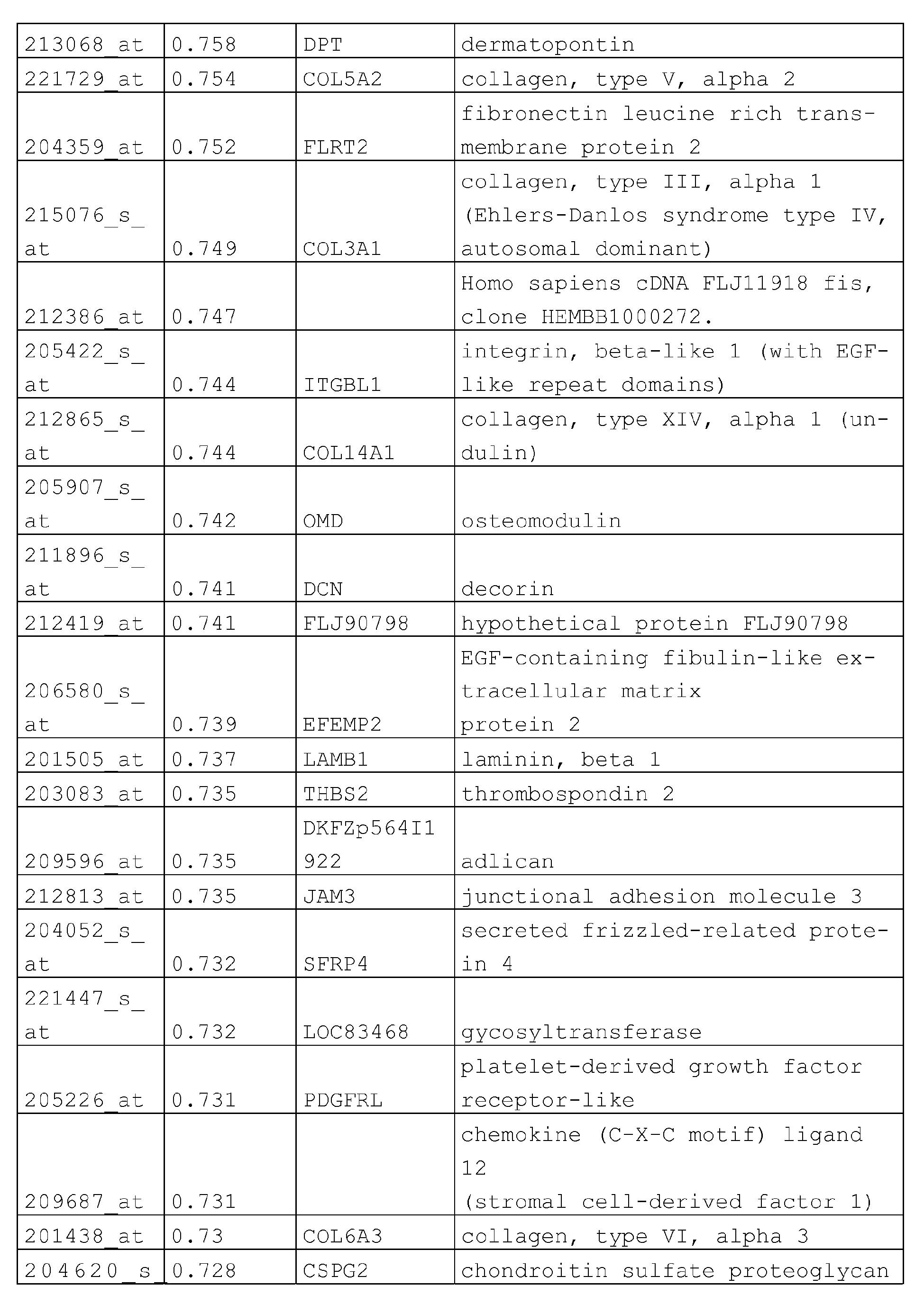

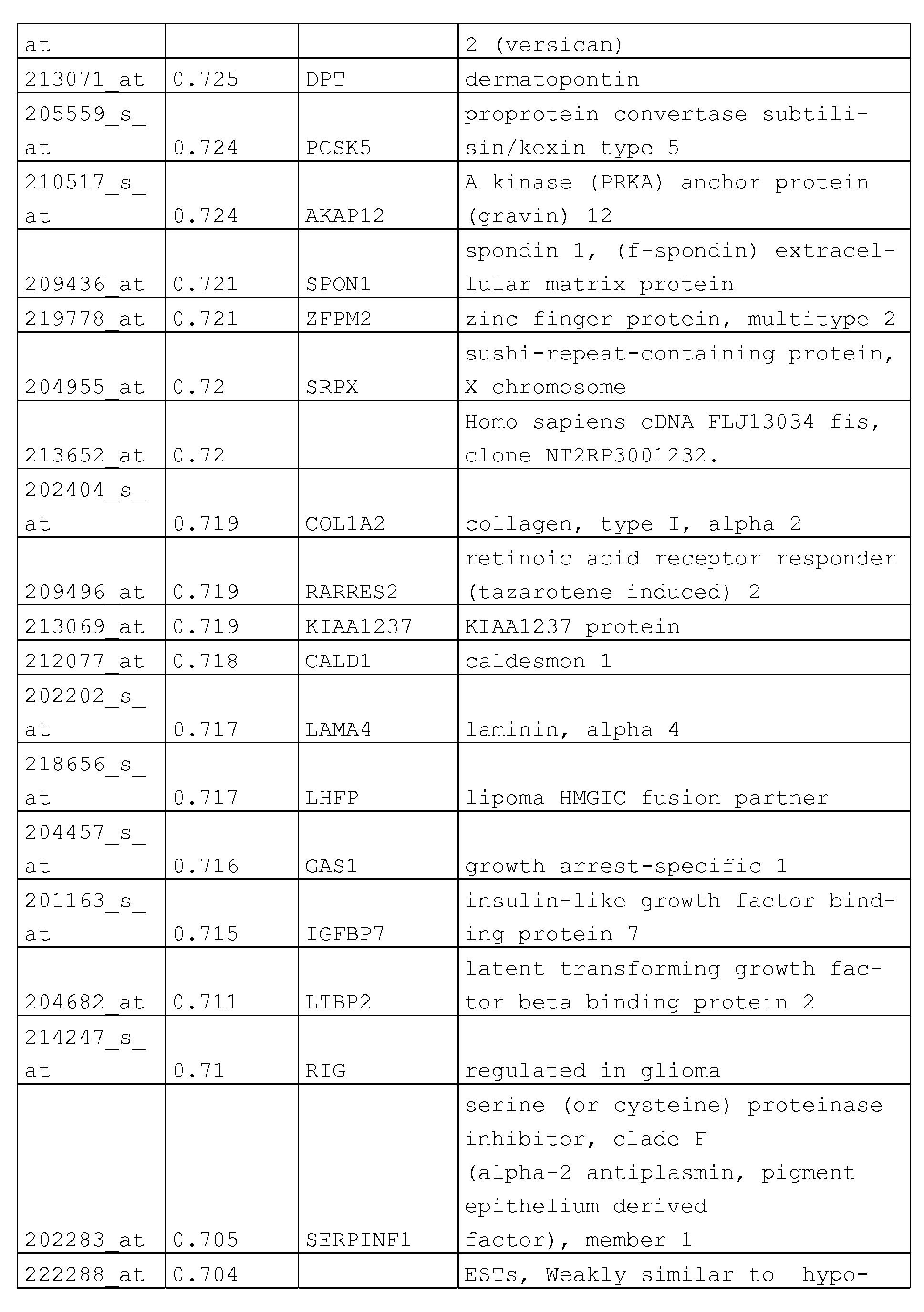

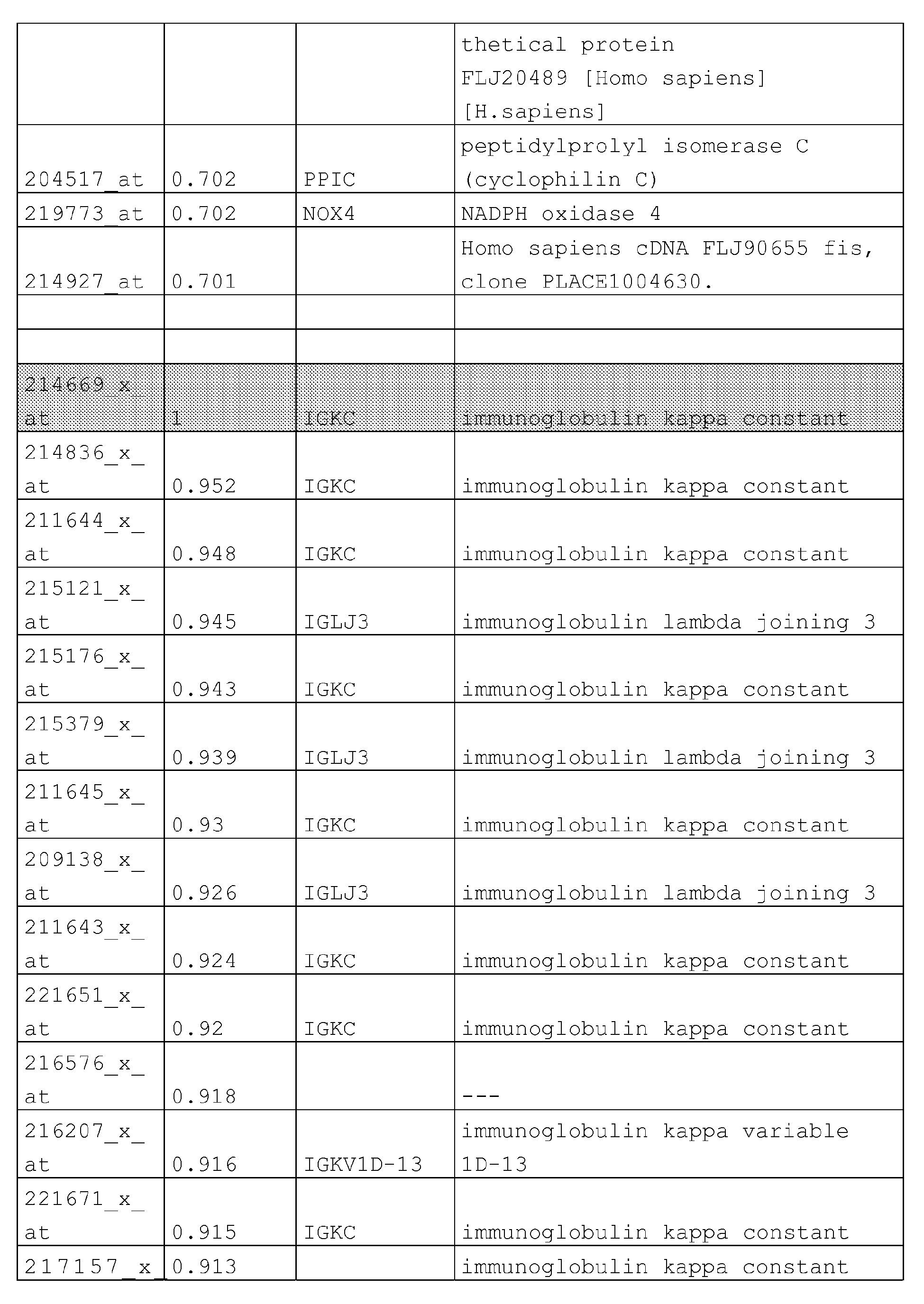

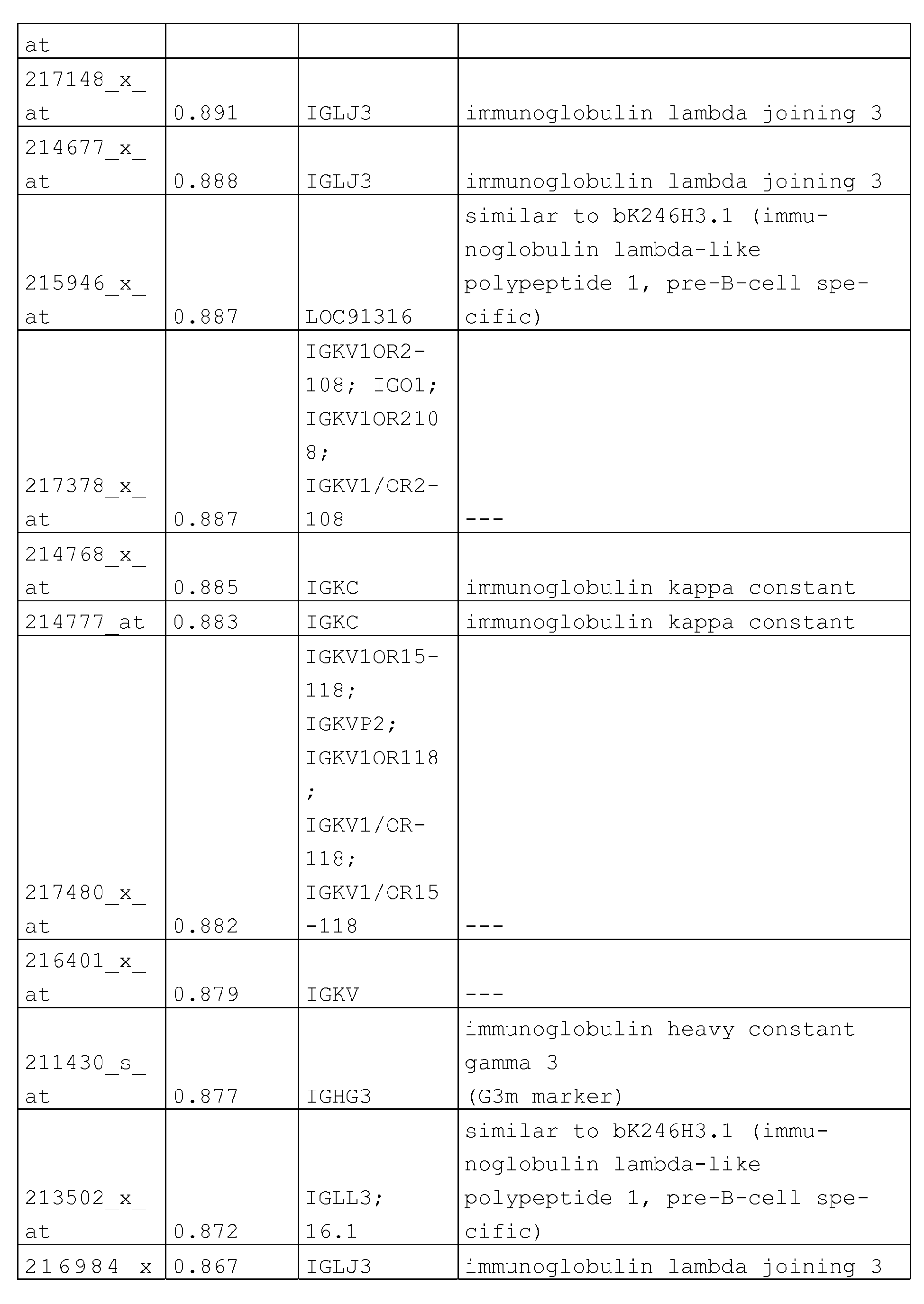

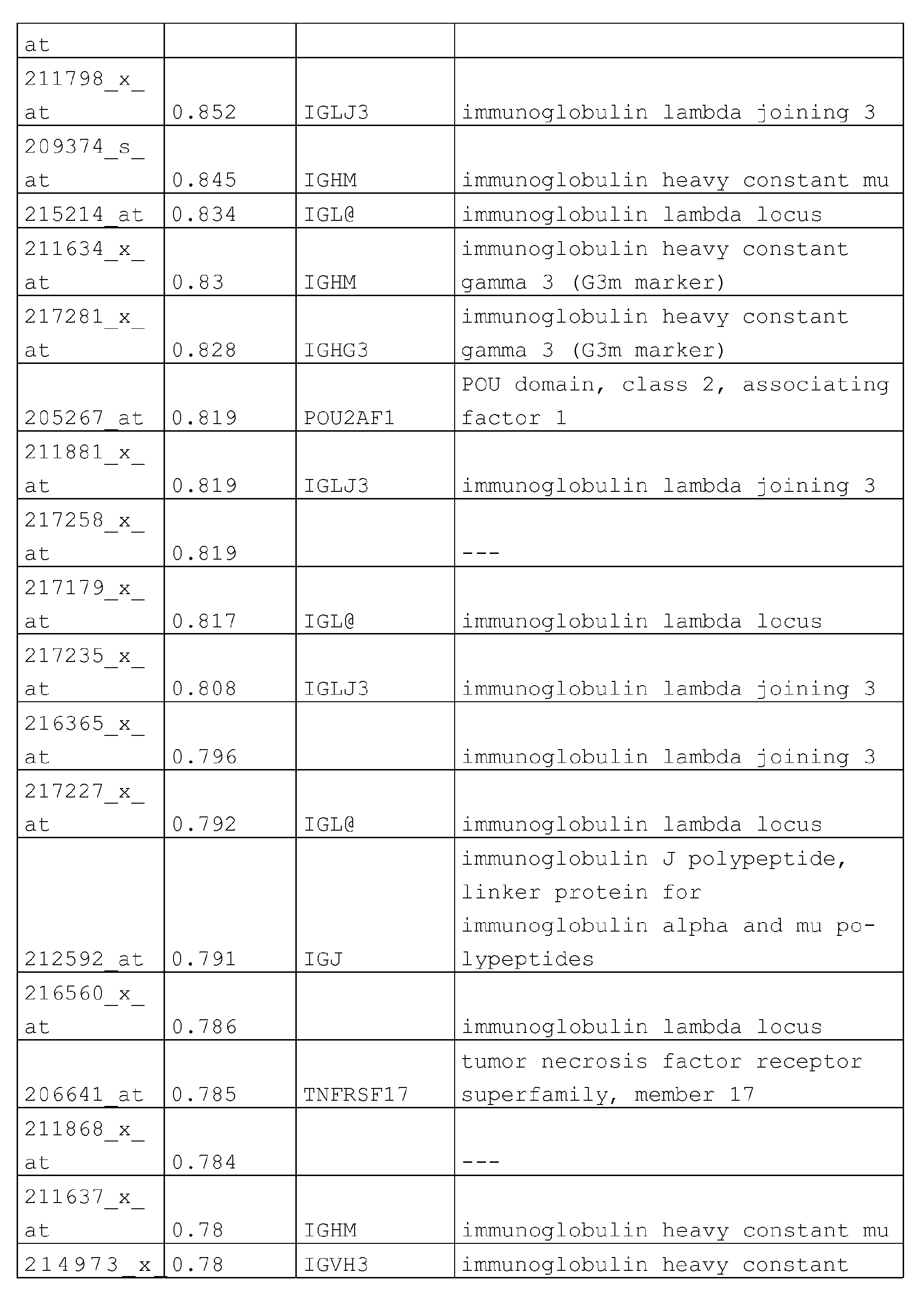

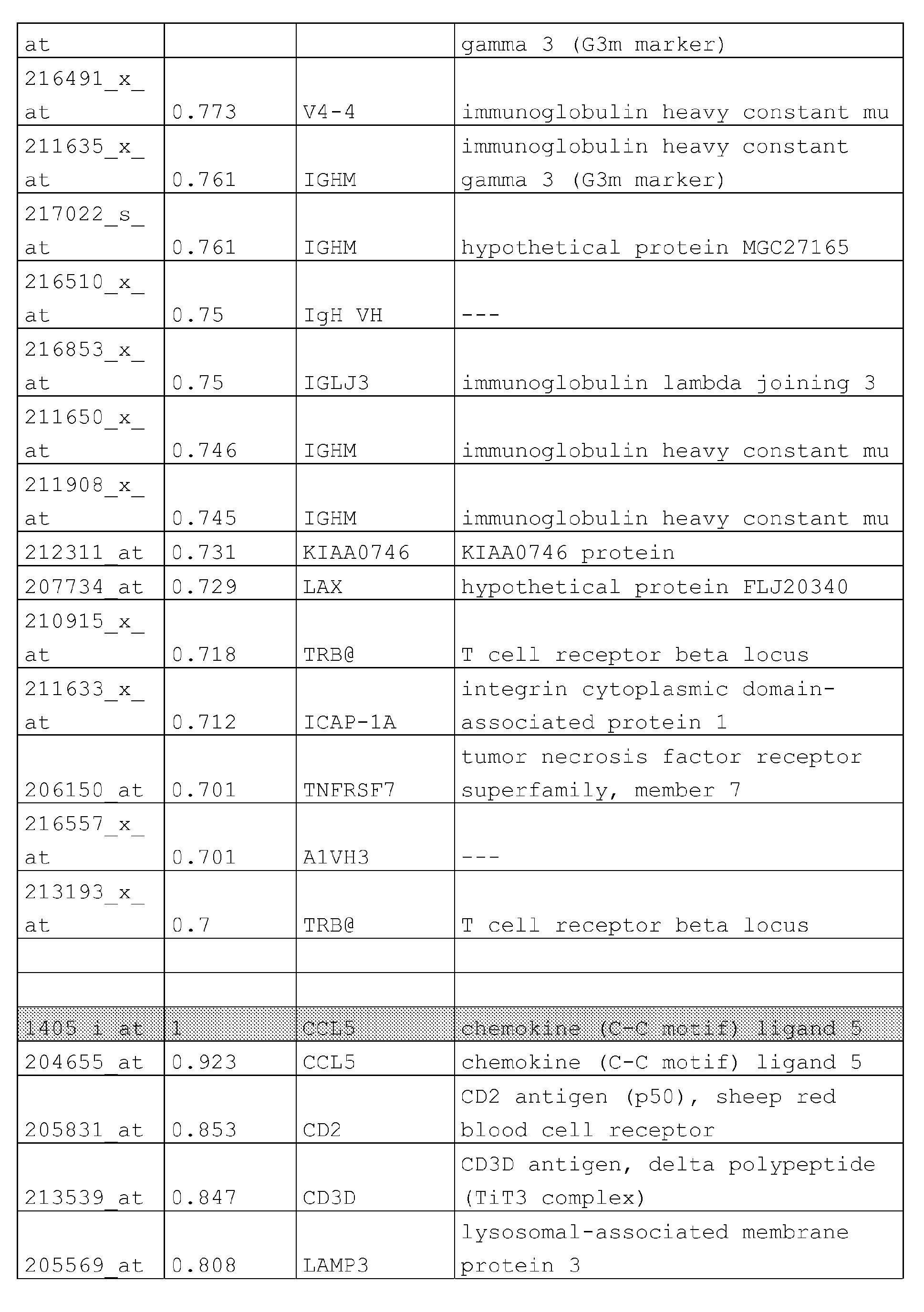

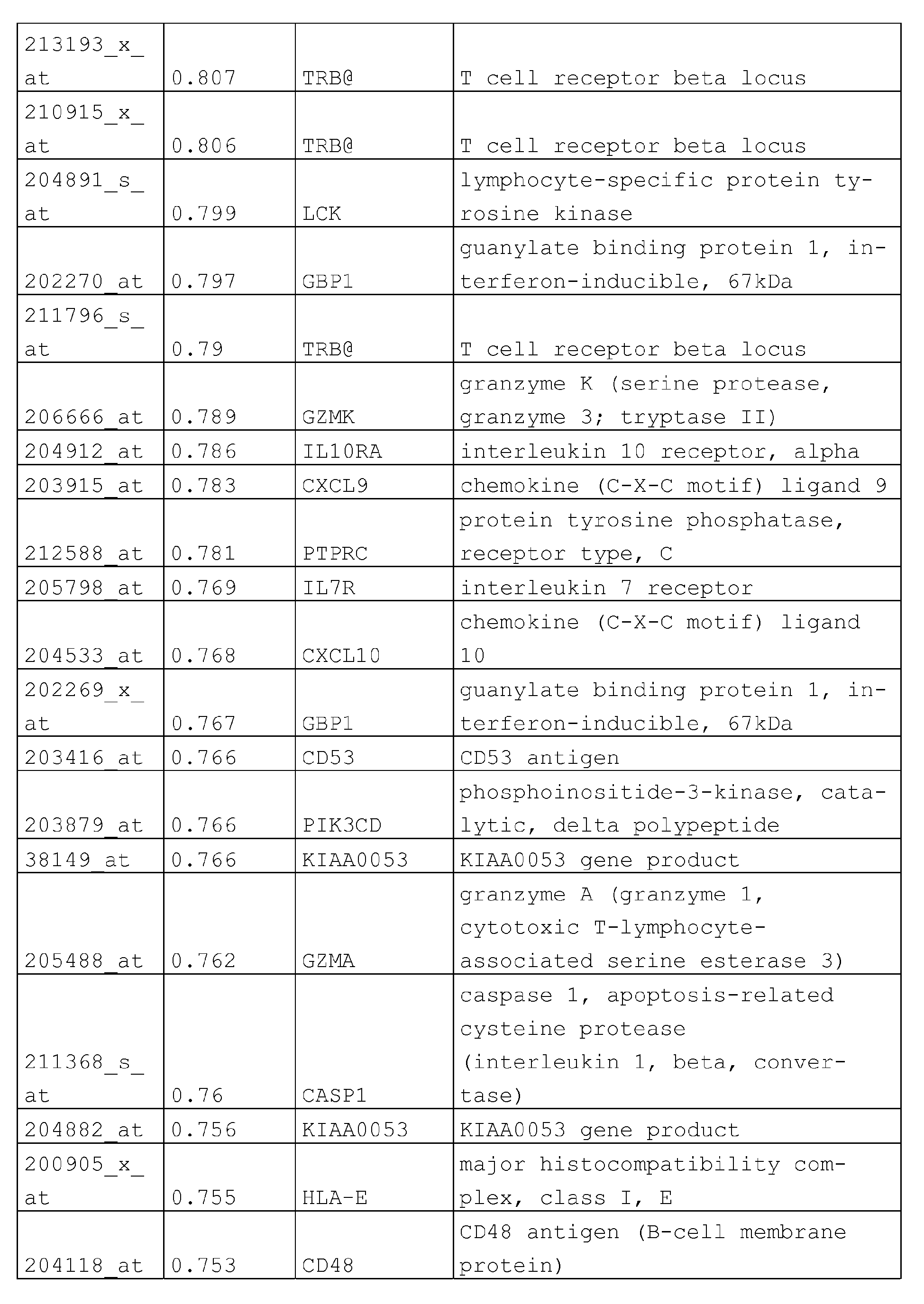

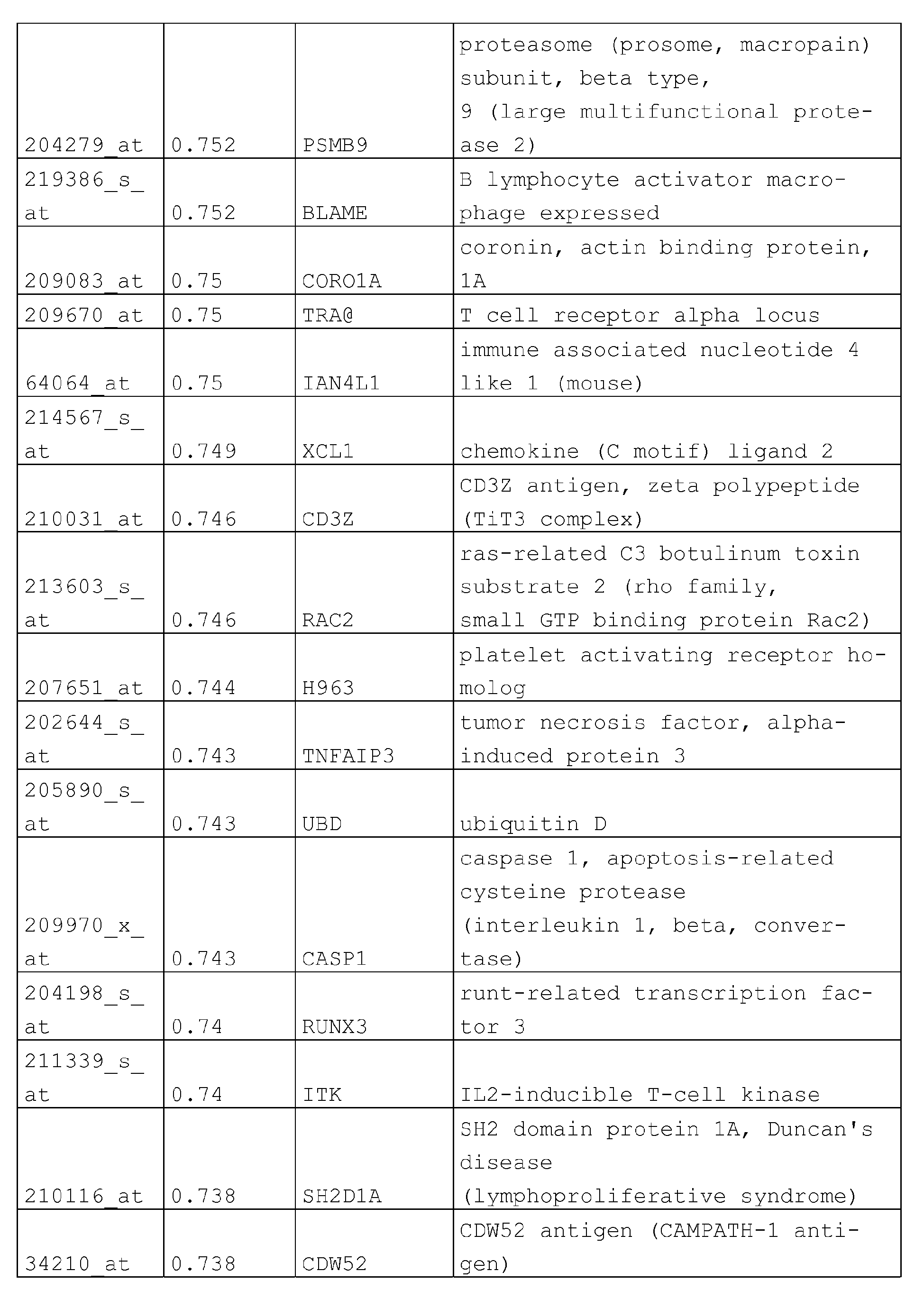

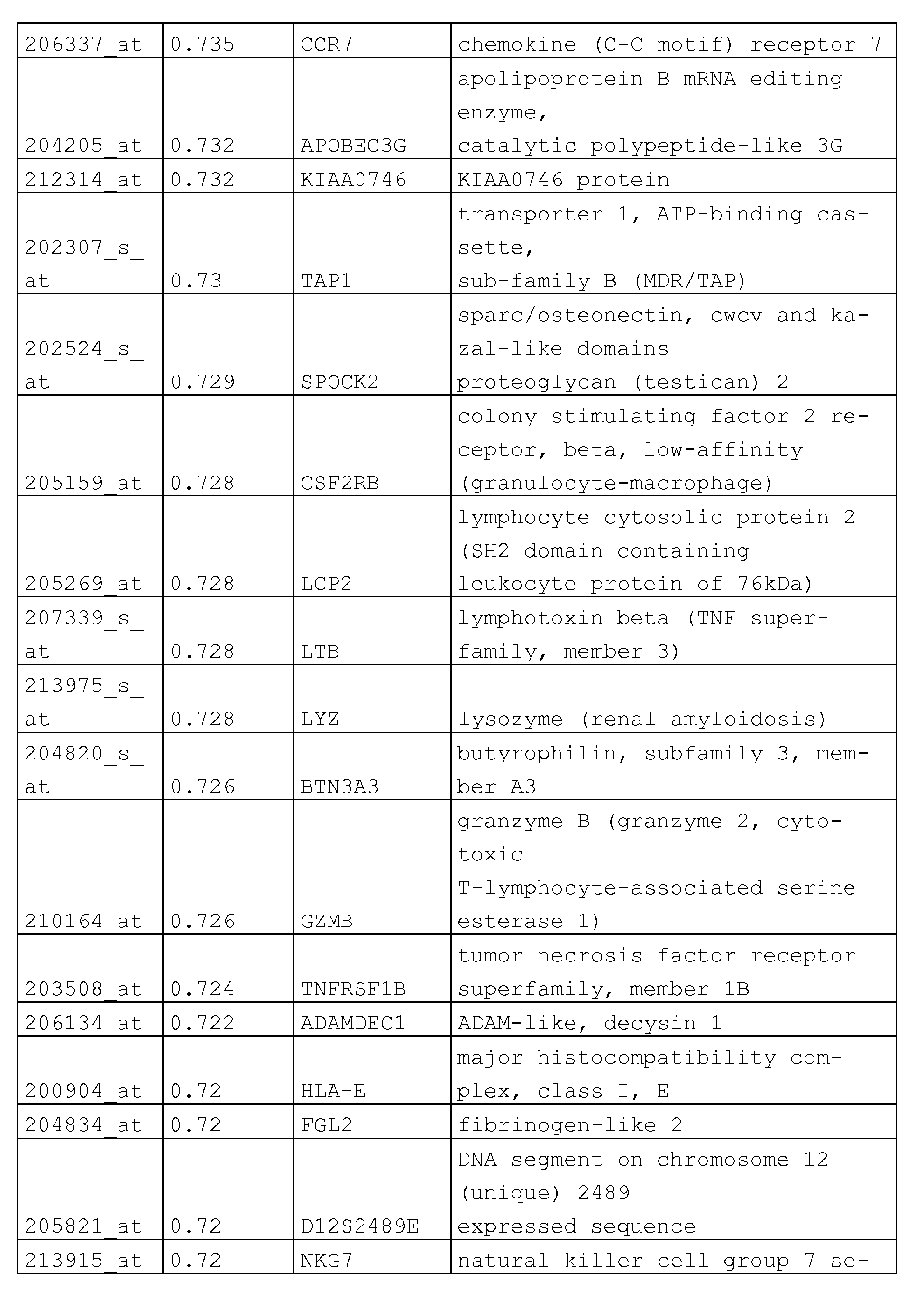

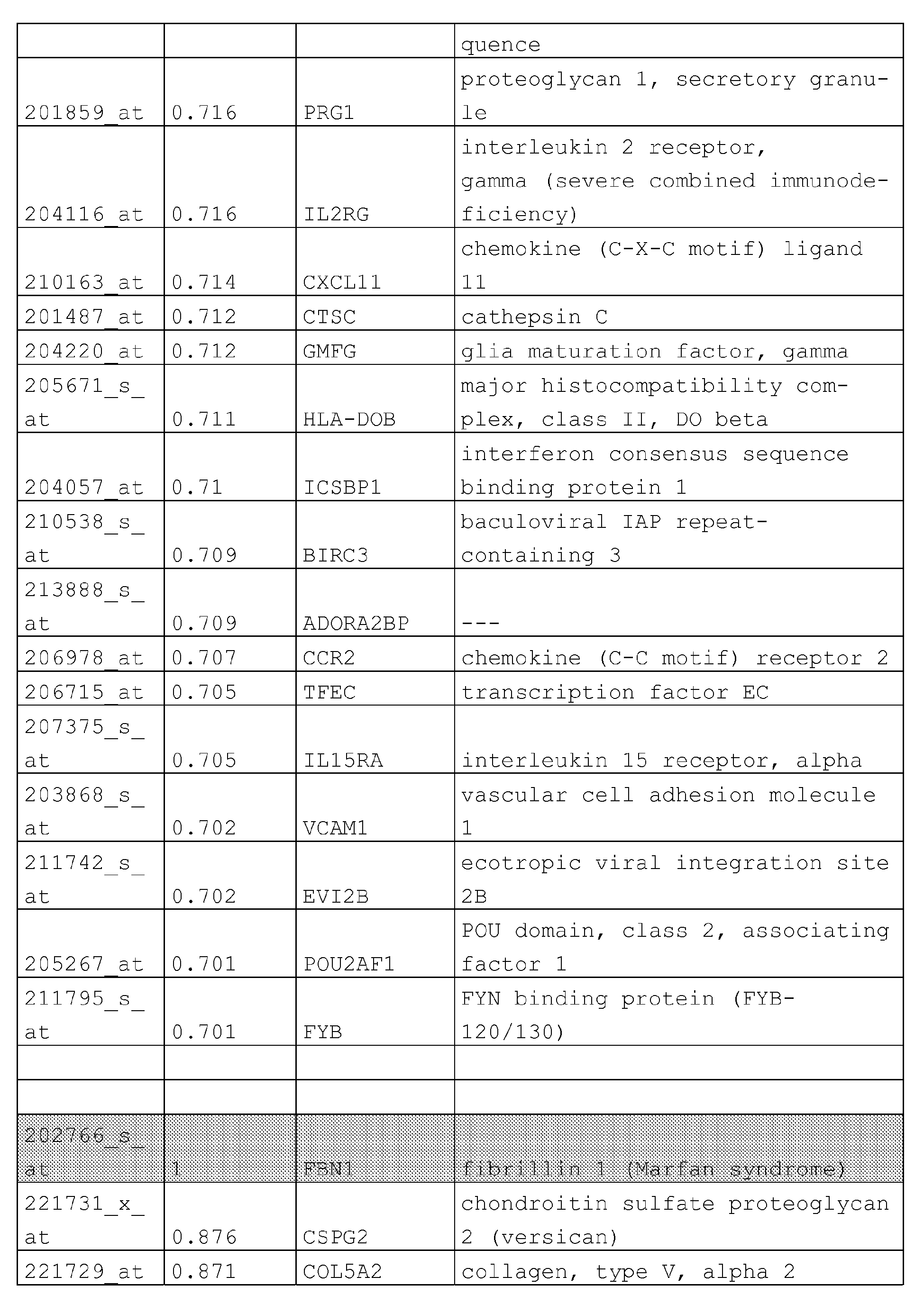

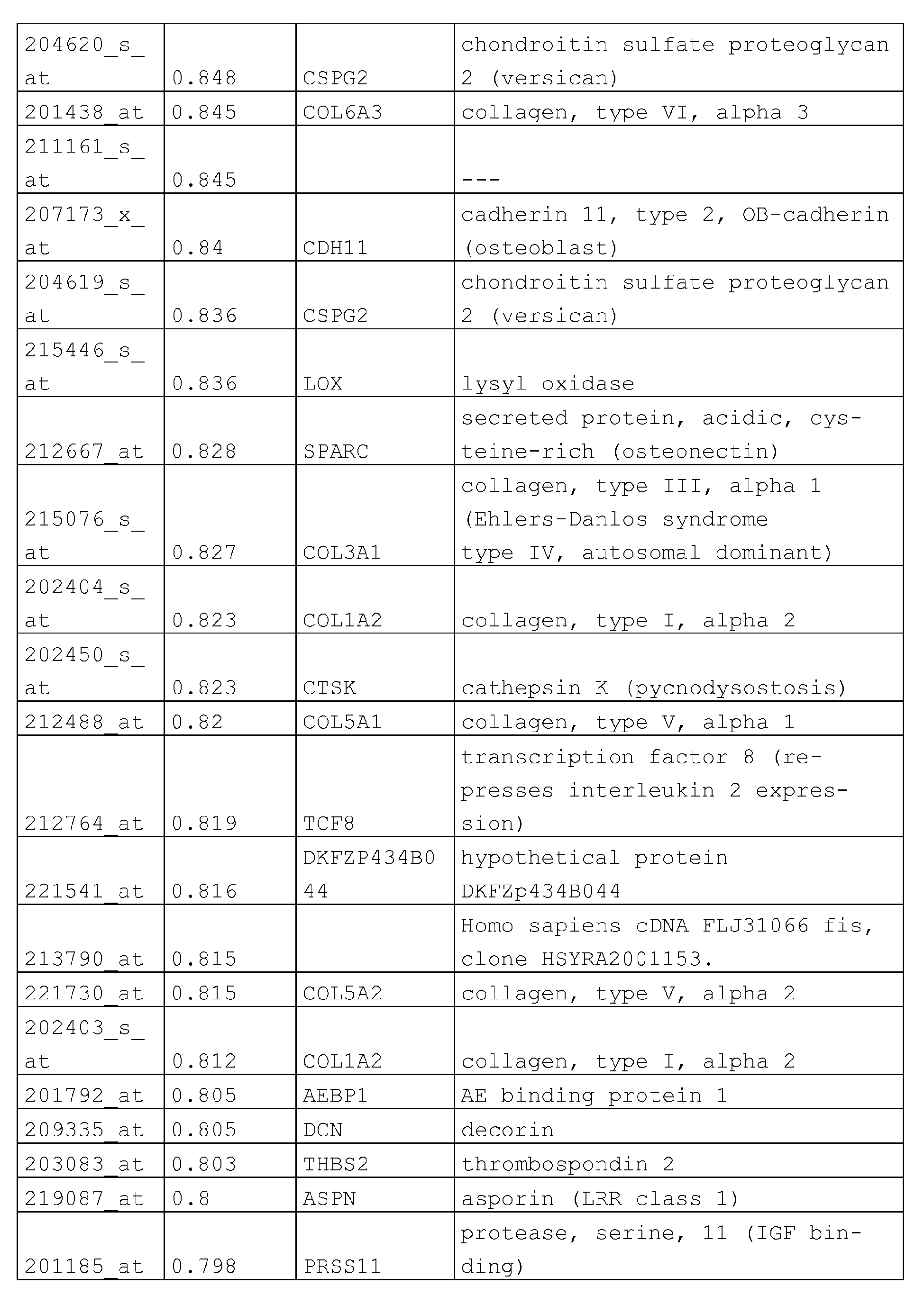

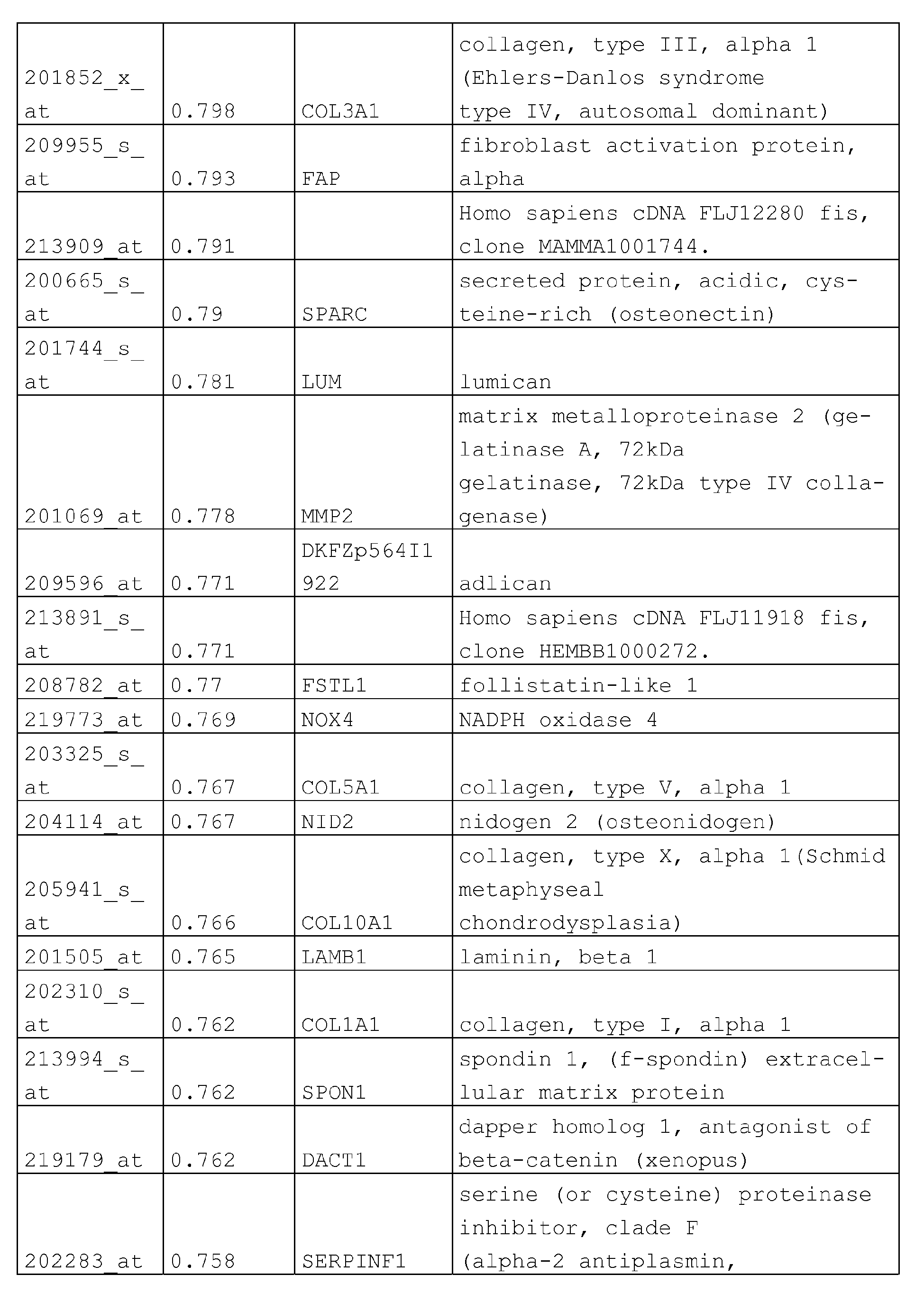

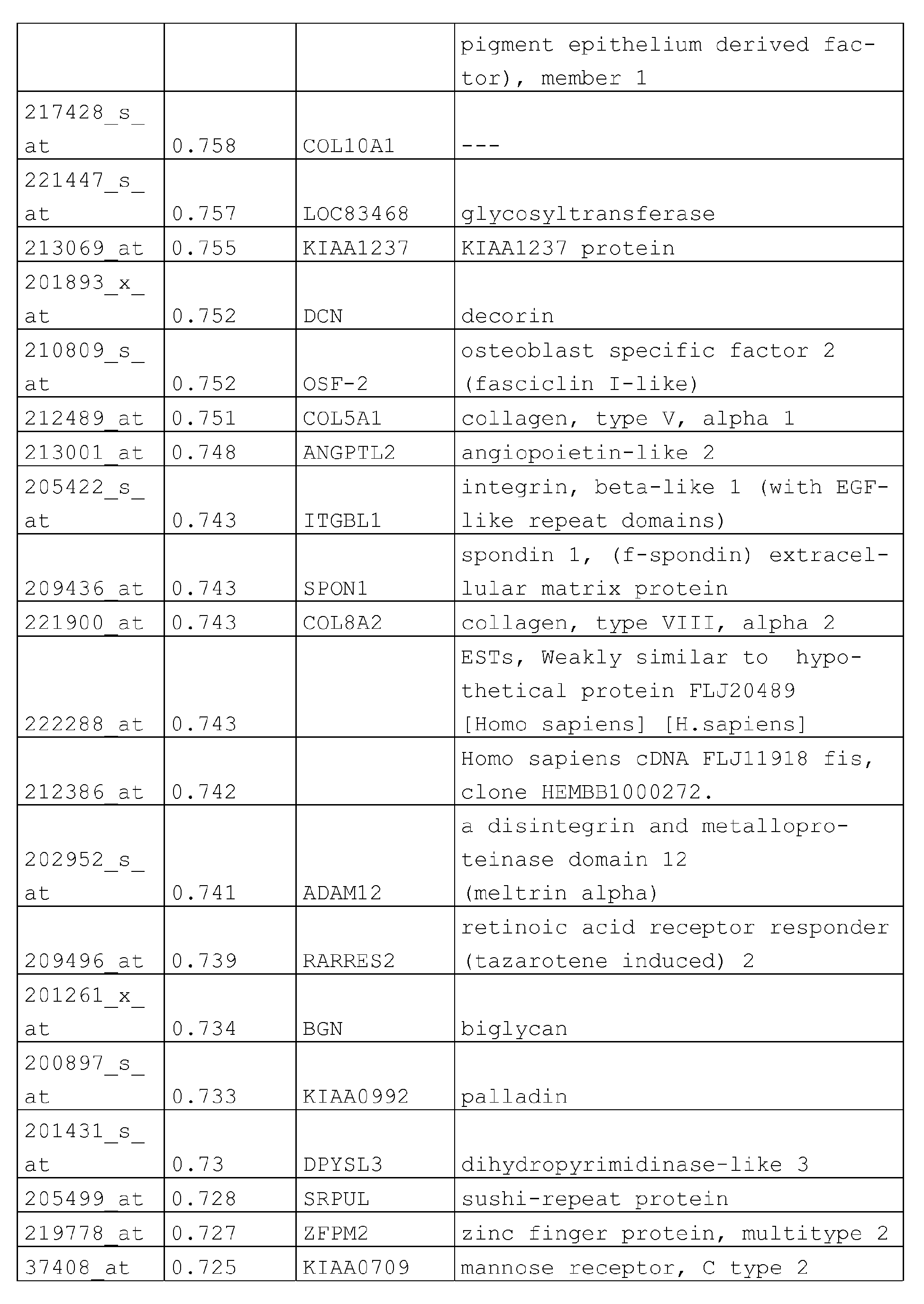

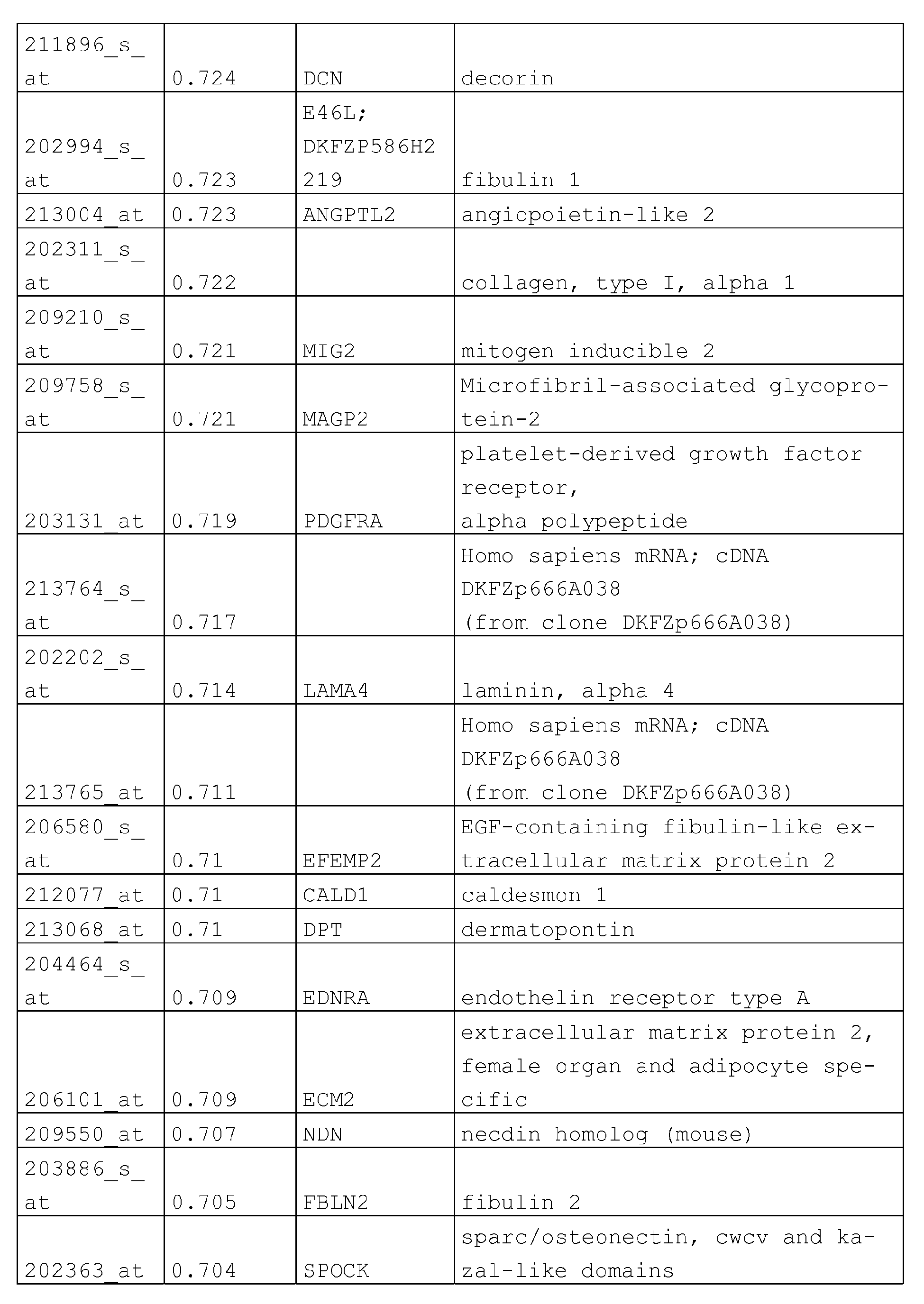

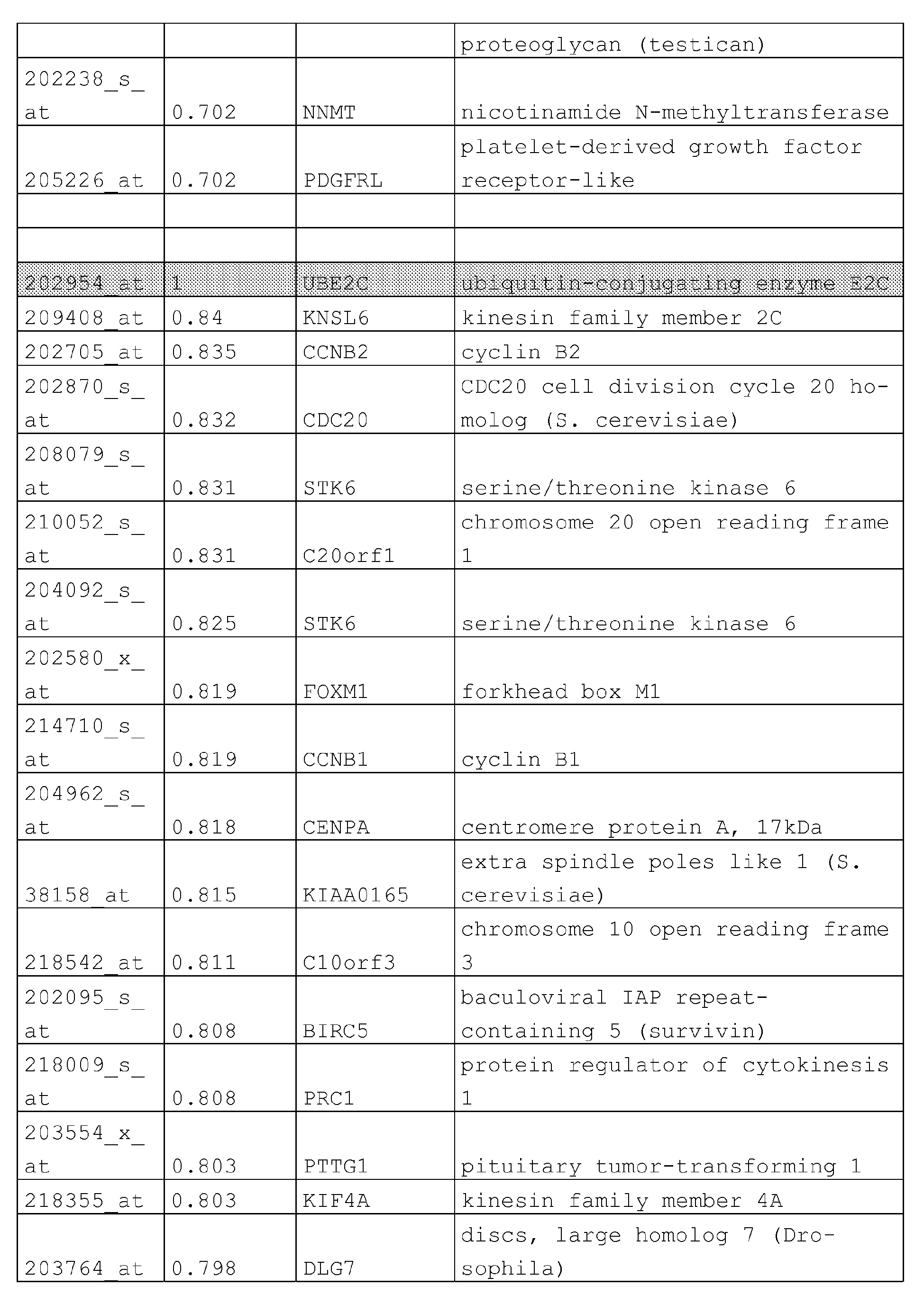

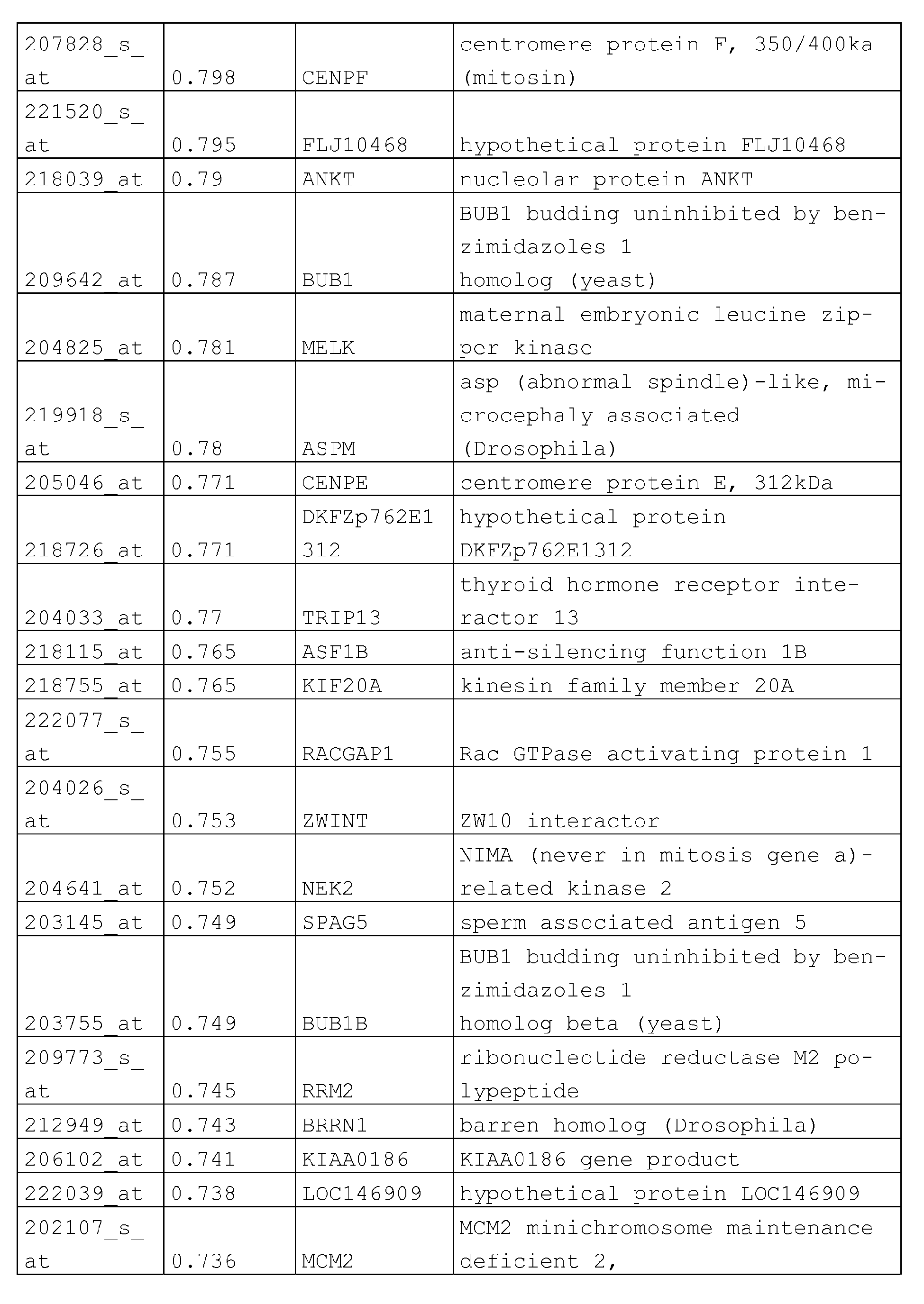

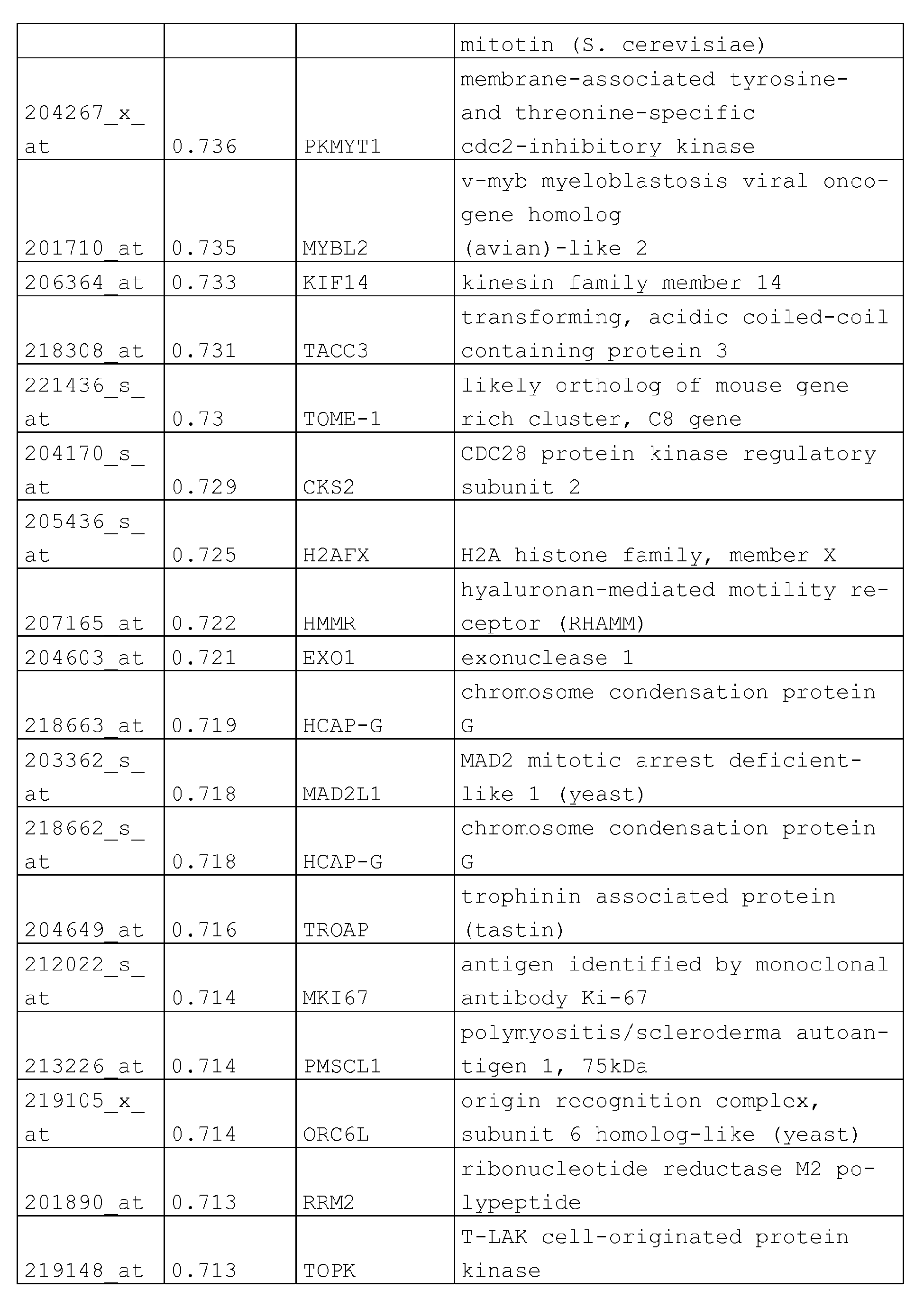

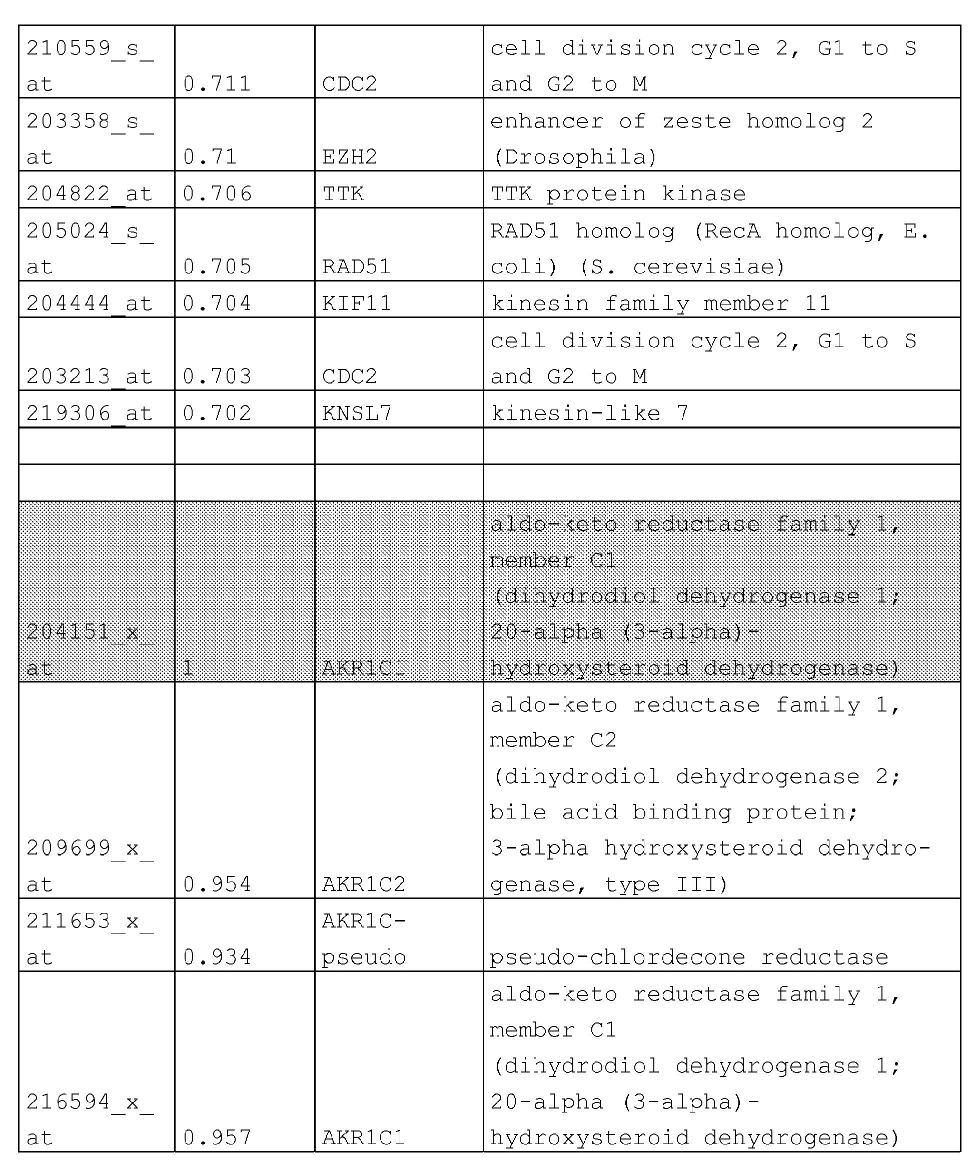

However, other genes not mentioned in the above table, which are yet coregulated with at least one of the genes enumerated under item b), do also fall under the scope of the present invention. The teaching presented herein will make it obvious to the person skilled in the art to find such coregulated genes in literature, in databases or the like, without the need of inventive step.

In a preferred embodiment, said method comprises the further steps of
g) selecting at least one chemotherapeutic agent which is predicted to be successful,
wherein the chemotherapeutic agent is selected from the group comprising Epirubicin, Paclitaxel, 5-Fluorouracil and/or Carboplatin.

The chemotherapeutics may be selected from the group consisting of Cyclophosphamid (Endoxan®, Cyclostin®). Adriamycin (Doxorubicin) (Adriblastin®), BCNU (Carmustin) (Carmubris®), Busulfan (Myleran®),Bleomycin (Bleomycin®), Carboplatin (Carboplat®), Chlorambucil (Leukeran®), Cis-Platin (Cisplatin®), Platinex (Platiblastin®), Dacarbazin (DTIC®;Detimedac®), Docetaxel (Taxotere®), Epirubicin (Farmorubicin®), Etoposid (Vepesid®), 5-Fluorouracil (Fluroblastin®, Fluorouracil®), Gemcitabin (Gemzar®), Ifosfamid (Holoxan®), Interferon alpha (Roferon®), Irinotecan (CPT 11, Campto®), Melphalan (Alkeran®),Methotrexat (Methotrexat®, Farmitrexat®), Mitomycin C (Mitomycin®), Mitoxantron (Novantron®), Oxaliplatin (Eloxatine®), Paclitaxel (Taxol®), Prednimustin (Sterecyt®), Procarbazin (Natulan®), Ralitrexed (Tomudex®), Trofosfamid (Ixoten®), Vinblastin (Velbe®), Vincristin (Vincristin®), Vindesin (Eldisine®), Vinorelbin (Navelbine®).

In a first step for the identification of an optimal predictive gene signature for single chemotherapeutic agents four different molecular subtypes (basal, luminal A, stromal-high and stromal-low) whose tumors differ in their response towards chemotherapy have been identified via the identified marker genes MLPH, ESR1, PGR and COMP.

The basal subtype is
- hormone receptor negative,
- progesterone receptor negative,
- Her2 receptor negative,
- sensitive towards most of the chemotherapeutic agents, and
- exhibits the gene MLPH as differentially expressed gene (down-regulated) and marker gene.

The luminal A subtype is
- resistant towards most of the chemotherapeutic agents, and
- exhibits the genes ESR1 and PGR as differentially expressed genes (up-regulated) and marker gene.
   The stromal-high subtype is
- resistant towards most of the chemotherapeutic agents, and
- exhibits the gene COMP as differentially expressed gene (up-regulated) and marker gene.
   The stromal-low subtype is
- sensitive towards most of the chemotherapeutic agents, and
- exhibits the gene COMP as differentially expressed gene (down-regulated) and marker gene.

In a second step for the identification of an optimal predictive gene signature *in vitro* chemosensitivity assays of primary tumors are performed to determine the response of a tumor towards a single chemotherapeutic agent. In said *in vitro* chemosensitivity assays the primary tumors were cultivated in different assays with increasing concentrations of the agents. After 6 days of incubation the vitality of the tumor cells were determined with an ATP-measurement. Hereby, the growing inhibition for the different agent concentrations could be determined and a dose-response curve could be provided. For each tumor the Area under the dose-response curve (AUC) could be determined for the different agents. The AUC is used to evaluate the response of a tumor towards a chemotherapeutic agent. The bigger the AUC, the more sensitive is the tumor towards the agent. The tumor samples were classified according to their sensitivity towards the agents into three classes (resistant, intermediate, sensitive) via the tertiles of the AUC arrangement.

For the expression analyses isolated RNA from the tumor tissues was used for molecular profiling with microarrays. Unsupervised hierarchical clustering and principal component analysis identified the molecular subtypes.

In a quantitative diagnostic assay it is a common practice to use a cut-off score which distinguishes between two different test results (up-regulated expression and down-regulated expression). The following cut off values relate to gene expression values determined by HG-U133a arrays of Affymetrix using MAS5.0 software with target intensity settings of 500.

Tumor tissues with an expression MLPH < 2000 (cut-off score) have been characterized as the basal molecular subtype. In case the expression of the genes ESR1 and PGR were > 6000 and 160, respectively, the tumor tissues have been characterized as the subtype luminal A. The remaining tumor tissues have been divided into two different subtypes via the stromal gene COMP (cut-off score 300).

The response of these molecular subtypes differs significantly towards single agents.

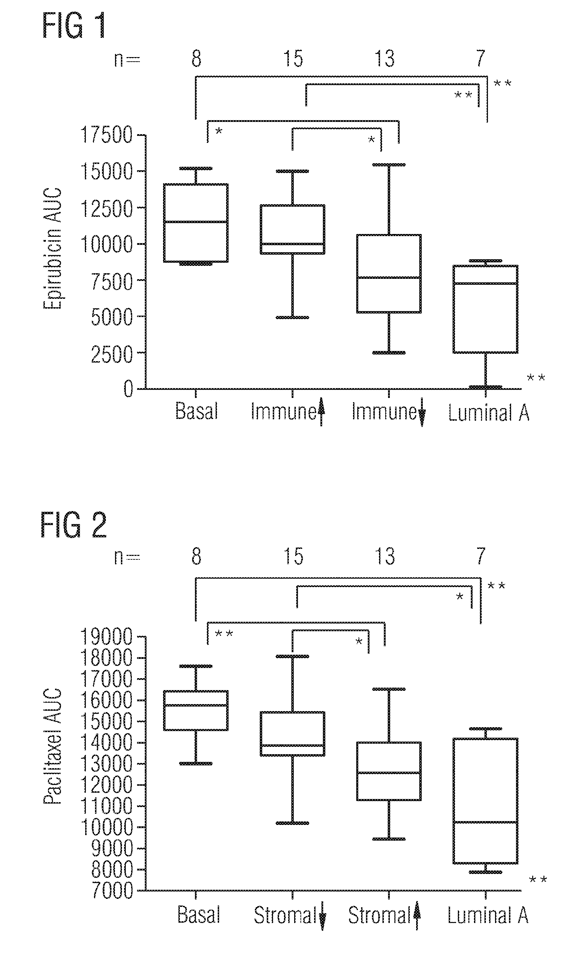

To improve separation between resistant and sensitive tumors for Epirubicin, Paclitaxel, 5-Fluorouracil and Carboplatin, additional biological motives were included in the analysis. Predictive gene signatures were defined for the single agents (Fig. 1 - 4). See Table 2 for an overview regarding the different molecular subtypes and their predictive gene expression signatures.

**Table 2: Overview regarding the different molecular subtypes and their predictive gene expression signatures**

| | MLPH | ESR1 | PGR | COMP | DCN | IGKC | CCL5 | FBN1 | UBE2C |
|---|---|---|---|---|---|---|---|---|---|
| basal | ↓ | ↓ | ↓ | | | | | | |
| stromal ↑ | | | | ↑ | ↑ | | | ↑ | |
| stromal ↓ | | | | ↓ | ↓ | | | ↓ | |
| luminal A | | ↑ | ↑ | | | | | | |
| immune system↑ | | | | | | ↑ | ↑ | | |
| immune system↓ | | | | | | ↓ | ↓ | | |
| proliferation ↑ | | | | | | | | | ↑ |
| proliferation ↓ | | | | | | | | | ↓ |

| | | | | | | | | | |
|---|---|---|---|---|---|---|---|---|---|
| + positive - negative ↑ up-regulated ↓ down-regulated | | | | | | | | | |

In a preferred embodiment
a) the basal molecular subtype predictive for Epirubicin sensitivity is characterized by down-regulated MLPH-expression, the luminal A molecular subtype predictive for Epirubicin resistance is characterized by up-regulated ESR1 and PGR expression, the immune system-high molecular subtype predictive for Epirubicin sensitivity is characterized by up-regulated IGKC and CCL5 expression, and/or the immune system-low molecular subtype predictive for Epirubicin resistance is characterized by down-regulated IGKC and CCL5 expression;
b) the basal molecular subtype predictive for Paclitaxel sensitivity is characterized by down-regulated MLPH expression, the luminal A molecular subtype predictive for Paclitaxel resistance is characterized by up-regulated ESR1 and PGR expression, the stromal-low molecular subtype predictive for Paclitaxel sensitivity is characterized by down-regulated DCN expression, and/or the stromal-high molecular subtype predictive for Paclitaxel resistance is characterized by up-regulated DCN expression;
c) the stromal-low molecular subtype predictive for 5-Fluorouracil sensitivity is characterized by down-regulated FBN1 expression, and/or the stromal-high molecular subtype predictive for 5-Fluorouracil resistance is characterized by up-regulated FBN1 expression; and/or
d) the stromal/proliferation-low molecular subtype predictive for Carboplatin sensitivity is characterized by down-regulated FBN1/UBE2C expression ratio, and/or the stromal/proliferation-high molecular subtype predictive for Carboplatin resistance is characterized by up-regulated FBN1/UBE2C expression ratio.

Since the tumors of the basal and luminal A subtype are particularly sensitive and resistant, respectively, towards the chemotherapeutic agents Epirubicin and Paclitaxel, the marker genes MLPH and ESR1 and PGR, respectively, are used for the prediction towards said agents.

In favor of the stromal subtypes the marker gene DCN is used for the prediction towards Paclitaxel (p=0.0188) (Fig. 2).

For the agent Epirubicin the tumors, which can not be classified as luminal A and basal subtypes, are divided via the expression average of the immune system genes IGKC (normalized B-cell) and CCL5 (normalized T-cell) into a resistant and sensitive group (p=0.0341) (Fig. 1). In case of signal intensities obtained by MAS5.0 software from HG-U133a arrays using scaling to a target intensity of 500, IGKC values are divided by 2338 and CCL5 values are divided by 399.5, corresponding to their respective median expression values in a reference cohort. In order to get an immune system score, both normalized values are added and divided by two.

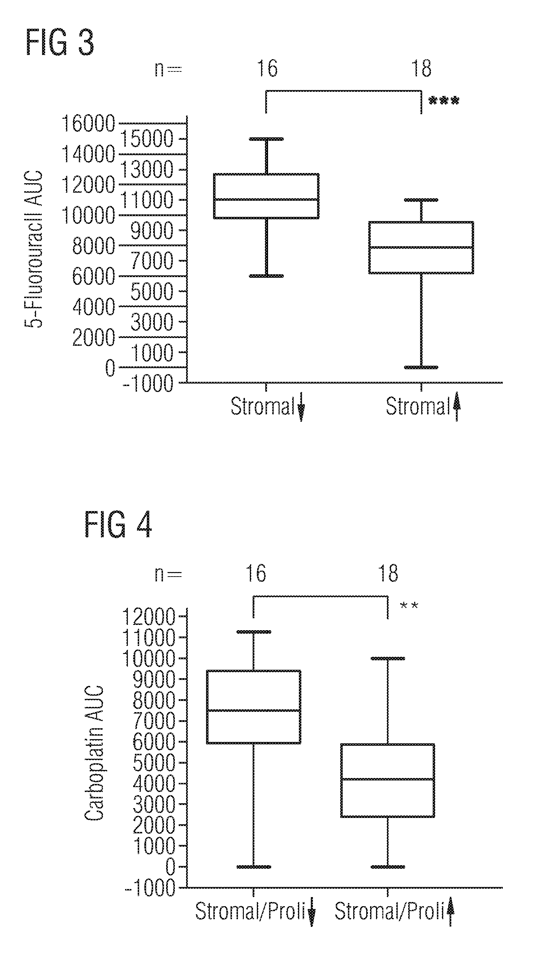

For 5-Fluorouracil all tumors are divided into two groups via the stromal gene FBN1 (p=0.0005) (Fig. 3). And regarding Carboplatin the proliferation gene UBE2C and the stromal gene FBN1 are used for classification. Due to the inverse correlation of said motives regarding chemosensitivity the ratio between UBE2C and FBN1 is calculated (Fig. 4). The tumors with high expressed proliferation genes and with low expressed stromal genes differ significantly (p=0.0029) to the tumors with an inverse ratio.

The defined cut-off score for MLPH predictive for Epirubicin is 2000, the defined score for ESR1 predictive for Epirubicin is 6000, the defined score for PGR predictive for Epirubicin is 160 and the defined score for the immune system score (average of IGKC and CCL5) predictive for Epirubicin is 1.5. An overview about the predictive gene (expression) signature for Epirubicin in breast cancer is demonstrated in Table 3.

**Table 3: Predictive gene (expression) signature for Epirubicin in breast cancer**

| | MLPH | ESR1 | PGR | IGKC + CCL5 |
|---|---|---|---|---|
| basal | ↓ | - | - | |
| luminal A | | ↑ | ↑ | |
| immune system↑ | | | | ↑ |
| immune system↓ | | | | ↓ |

| | | | | |
|---|---|---|---|---|
| + positive - negative ↑ up-regulated ↓ down-regulated | | | | |

The defined cut-off score for MLPH predictive for Paclitaxel is 2000, the defined score for ESR1 predictive for Paclitaxel is 6000, the defined score for PGR predictive for Paclitaxel is 160 and the defined score for DCN predictive for Paclitaxel is 1500. An overview about the predictive gene (expression) signature for Paclitaxel in breast cancer is demonstrated in Table 4.

**Table 4: Predictive gene (expression) signature for Paclitaxel in breast cancer**

| | MLPH | ESR1 | PGR | DCN | COMP |
|---|---|---|---|---|---|
| basal | ↓ | - | - | | |
| luminal A | | ↑ | ↑ | | |
| stromal ↑ | | | | ↑ | ↑ |
| stromal ↓ | | | | ↓ | ↓ |

| | | | | | |
|---|---|---|---|---|---|
| + positive - negative ↑ up-regulated ↓ down-regulated | | | | | |

The defined cut-off score for FBN1 predictive for 5-Fluorouracil is 3500. An overview about the predictive gene (expression) signature for 5-Fluorouracil in breast cancer is demonstrated in Table 5.

**Table 5: Predictive gene (expression) signature for 5-Fluorouracil in breast cancer**

| | FBN1 | COMP |
|---|---|---|
| stromal ↑ | ↑ | ↑ |
| stromal ↓ | ↓ | ↓ |

| | | |
|---|---|---|
| + positive - negative ↑ up-regulated ↓ down-regulated | | |

The defined cut-off score for the ratio between FBN1 and UBE2C predictive for Carboplatin is 1. An overview about the predictive gene (expression) signature for Carboplatin in breast cancer is demonstrated in Table 6.

**Table 6: Predictive gene (expression) signature for Carboplatin in breast cancer**

| | FBN1 / UBE2C |
|---|---|
| stromal / proliferation ↓ | ↓ |
| stromal / proliferation ↑ | ↑ |

| | |
|---|---|
| + positive - negative ↑ up-regulated ↓ down-regulated | |

In another preferred embodiment of the present invention, Paclitaxel resistance in the basal molecular subtype is characterized by up-regulated AKR1C1 expression. It is particularly preferred that, in the method according to the invention, the said expression level is determined by
a) a hybridization based method;
b) a PCR based method;
c) determining the protein level, and/or by
d) an array based method.

In yet another preferred embodiment of the present invention, it is provided that the expression level of at least one of the said genes is determined with rtPCR (reverse transcriptase polymerase chain reaction) of the gene related mRNA.

In another preferred embodiment of the present invention, it is provided that the expression level of at least one of the said genes is determined in formalin and/or paraffin fixed tissue samples.

Routinely, in tumor diagnosis tissue samples are taken as biopsies from a patient and undergo diagnostic procedures. For this purpose, the samples are fixed in formaline and/or parrafine and are then examined with immunohistochemistry methods. The formaline treatment leads to the inactivation of enzymes, as for example the ubiquitous RNA-digesting enzymes (RNAses). For this reason, the mRNA status of the tissue (the so called transcriptome), remains undigested.

However, the formaline treatment leads to partial depolymerization of the individual mRNA molecules. For this reason, the current doctrine is that formaline fixed tissue samples can not be used for the analysis of the transcriptome of said tissue.

For this reason, it is provided in a preferred embodiment of the present invention that after lysis, the samples are treated with silica-coated magnetic particles and a chaotropic salt, in order to purify the nucleic acids contained in said sample for further determination.

Collaborators of the inventors of the present invention have developed an approach which however allows successful purification of mRNA out of tissue samples fixed in such manner, and which is disclosed, among others, in W003058649, WO2006136314A1 and DE10201084A1, the content of which is incorporated herein by reference.

Said method comprises the use of magnetic particles coated with silica (SiO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields. These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidinisothiocyanate. Said binding properties are known as the so called "boom principle". They are described in the European patent EP819696.

The said approach is particularly useful for the purification of mRNA out of formaline and/or paraffine fixed tissue samples. In contrast to most other approaches, which leave very small fragments behind that are not suitable for later determination by PCR and/or hybridization technologies, the said approach creates mRNA fragments which are large enough to allow specific primer hybridzation and/or specific probe hybridization. A minimal size of at least 100 bp, more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead based technologies. This of particular importance, as the inventors have observed, that DNAse treatment is not efficient in approximately 10% of FFPE samples generated by standard procedures and stored at room temperature for some years before cutting and RNA extraction.

The said approach thus allows a highly specific determination of candidate gene expression levels with one of the above introduced methods, particularly with hybridization based methods, PCR based methods and/or array based methods, even in formaline and/or paraffine fixed tissue samples, and is thus extremely beneficial in the context of the present invention, as it allows the use of tissue samples fixid with formaline and/or paraffine, which are available in tissue banks and connected to clinical databases of sufficient follow-up to allow retrospective analysis.

However, other methods are appropriate for the purification of mRNA out of formaline and/or paraffine fixed tissue samples as well.

In a yet preferred embodiment of the present invention said gynecologic cancer is selected from the group comprising breast cancer, ovarian cancer, vulvar cancer, vaginal cancer, tubal cancer, endometrian cancer and/or cervical cancer.

In an especially preferred embodiment of the present invention said gynecologic cancer is breast cancer. The method according to the invention may be used for the analysis of a wide variety of neoplastic cell growth and proliferation of the breast tissues including, but not limited to ductal carcinoma in situ, lobular carcinoma, colloid carcinoma, tubular carcinoma, medullary carcinoma, metaplastic carcinoma, intraductal carcinoma in situ, lobular carcinoma in situ and papillary carcinoma in situ.

Furthermore, a kit useful for carrying out one of the said methods, comprising at least
a) a primer pair and/or a probe each having a sequence sufficiently complementary to a marker gene according to the present invention; and/or
b) at least an antibody directed against a marker according to the present invention is provided.

In yet another embodiment of the invention a method for correlating the clinical outcome of a patient suffering from or at risk of developing recurrent gynecologic cancer, preferably breast cancer, with the presence or non-presence of a defect in marker gene expression is provided, said method comprising the steps of:
a) obtaining a biological sample from said patient;
b) determining the expression level of at least one marker gene according to the present invention in said patient, and
c) correlating the pattern of expression levels determined in (b) with said patient's data, said data being selected from the group consisting of etiopathology data, clinical symptoms, anamnesis data and/or data concerning the therapeutic regimen.

In yet another embodiment of the invention a nucleic acid molecule is provided, said nucleic acid molecule selected from the group comprising
a) the nucleic acid molecule presented as SEQ ID NO: 1 - 30,
b) a nucleic acid molecule having a length of 4 - 80 nucleotides, preferably 18 - 30 nucleotides, the sequence of which corresponds to the sequence of a single stranded fragment of a gene encoding for a marker from the group comprising MLPH, ESR1, PGR, COMP, DCN, IGKC, CCL5, FBN1, UBE2C and/or AKR1C1,
c) a nucleic acid molecule that is a fraction, variant, homologue, derivative, or fragment of the nucleic acid molecule presented as SEQ ID NO: 1 - 30,
d) a nucleic acid molecule that is capable of hybridizing to any of the nucleic acid molecules of a) - c) under stringent conditions,
e) a nucleic acid molecule that is capable of hybridizing to the complement of any of the nucleic acid molecules of a) - d) under stringent conditions,
f) a nucleic acid molecule that is capable of hybridizing to the complement of a nucleic acid molecule of e),
g) a nucleic acid molecule having a sequence identity of at least 95 % with any of the nucleic acid molecules of a) - f),
h) a nucleic acid molecule having a sequence identity of at least 70 % with any of the nucleic acid molecules of a) - f),
i) a complement of any of the nucleic acid molecules of a) - h), and/or
j) a nucleic acid molecule that comprises any nucleic acid molecule of a) - i).

Genes of interest are listed in Table 7, and the sequence listing is depicted in Table 8. These nucleic acids are being used either as primers for a polymerase chain reaction protocol, or as detectable probes for monitoring the said process.

Furthermore it is provided that the said nucleic acid is selected from the group comprising DNA, RNA, PNA, LNA and/or Morpholino. The nucleic acid may, in a preferred embodiment, be labelled with at least one detectable marker. This feature is applicable particularly for those nucleic acids which serve as detectable probes for monitoring the polymerase chain reaction process

Such detectable markers may for example comprise at least one label selected from the group consisting of fluorescent molecules, luminescent molecules, radioactive molecules, enzymatic molecules and/or quenching molecules.

In a particularly preferred embodiment, the said detectable probes are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

In another preferred embodiment of the present invention, a kit of primers and/or detection probes is provided, comprising at least one of the nucleic acids according to the above enumeration and/or their fractions, variants, homologues, derivatives, fragments, complements, hybridizing counterparts, or molecules sharing a sequence identity of at least 70%, preferably 95 %.

Said kit may, in another preferred embodiment, comprise at least one of the nucleic acid molecules presented as SEQ ID NO: 1 - 30, and/or their fractions, variants, homologues, derivatives, fragments, complements, hybridizing counterparts, or molecules sharing a sequence identity of at least 70%, preferably 95 %, for the detection of at least one marker gene according to the present invention.

Furthermore, the use of a nucleic acid according as recited above, or of a kit as recited above for the prediction of a clinical response of a patient suffering from or at risk of developing recurrent gynecologic cancer, preferably breast cancer, towards a chemotherapeutic agent is provided.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

### Example 1

The predictive gene signatures for the chemotherapeutic agents Epirubicin, 5-Fluorouracil and Paclitaxel have been validated in neoadjuvant studies via the defined cut-off scores. The Epirubicin prediction markers have been tested, to which extent they can predict the relative tumor reduction towards a neoadjuvant EC (Epirubin, Cyclophosphamid) combination therapy in 86 patients. Furthermore, the predictive genes for Epirubicin and 5-Fluorouracil were tested in a study, in which 39 patients received a neoadjuvant FEC (5-Fluorouracil, Epirubicin and Cyclophosphamid) therapy.

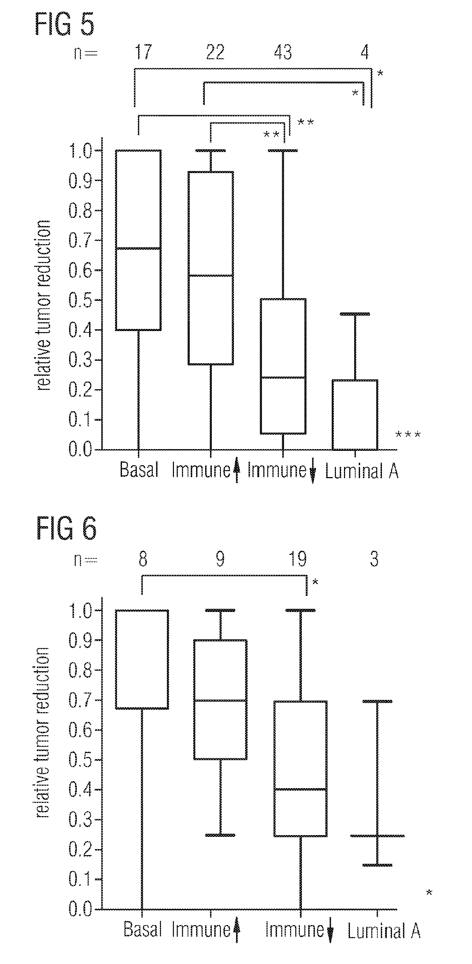

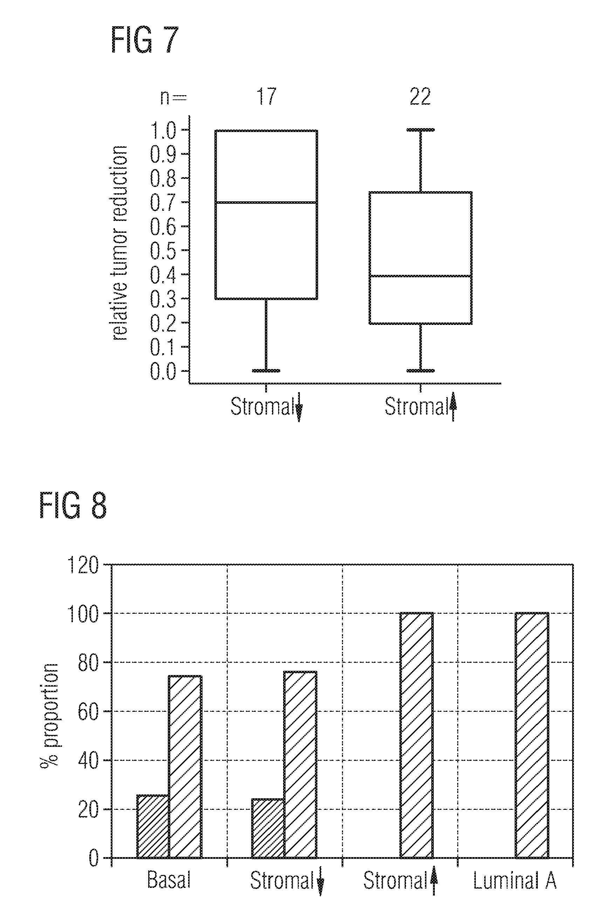

Via the predictive gene signature for Epirubicin the tumors of the neoadjuvant studies were divided into four prediction classes. Most of the tumors being sensitive towards the chemotherapeutic agents were classified as basal subtype and the class with a high expression of the immune system genes. The resistant tumors can be mainly found in the luminal A subtype and in the group with a low expressed immune system. In the EC study the four molecular prediction classes differ among each other significantly (p=0.0008). In particular noticeable is the difference, which occurs for the classification with the immune system markers (p=0.0038) (Fig. 5). In the classification of the FEC study with the Epirubicin prediction markers only the basal subtype and the group with the low expression of the immune system markers diverged significantly (p=0.0207). In contrast, between the two immune system subtypes there is a trend to statistical significance (p=0.0848) (Fig. 6). The 5-Fluorouracil prediction markers divide the tumors into two prediction classes, which differ in regard to the tumor reduction with a trend to statistical significance (Fig. 7).

For the validation of the predictive Paclitaxel gene signatures two different neoadjuvant studies could be used, wherein the clinical response towards TAC (Rody et al., 2006) ²¹ and T-FAC combination (Hess et al., 2006) ¹⁸ was analyzed. Since no data have been provided for the percental tumor reduction, it could be only performed a classification via the provided clinical information. For each prediction class of the Paclitaxel prediction markers the percentage of the (pathological) complete and incomplete remission (pCR/CR), respectively, has been determined.

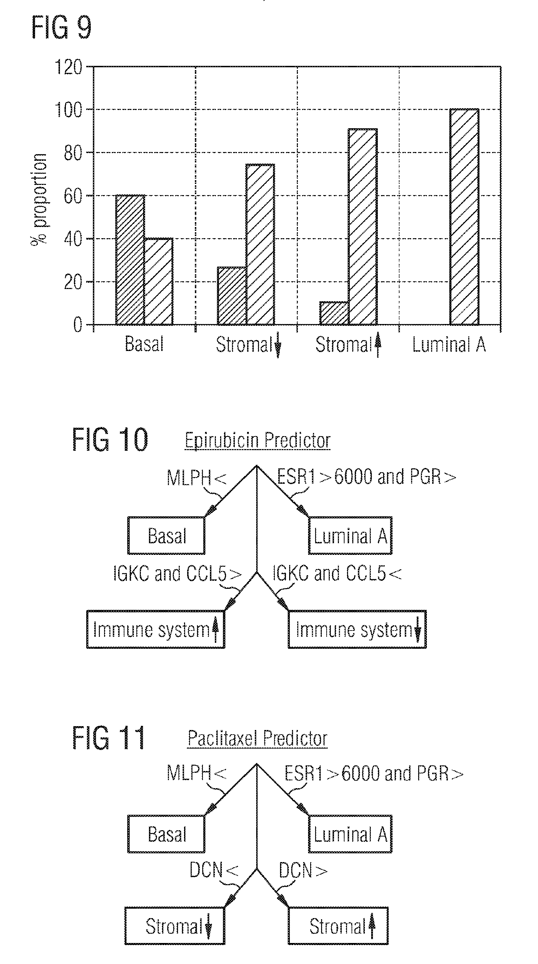

In the neoadjuvant TAC study with the Paclitaxel marker genes all patients with a complete remission (in each case the bar on the left side) were classified into the basal subtype and the subtype with low expression of the stromal motive, respectively (Fig. 8). In the second validation study most of the tumors showing a pathological complete remission (in each case the bar on the left side) towards a T-FAC combination therapy have been classified into these two subtypes as well. A little percentage has been classified into the subtype with a high expression of the stromal genes (Fig. 9).

### Discussion

The inventors of the present invention described for the first time methods for predicting the response of a tumor in a patient suffering from or at risk of developing recurrent breast cancer towards single chemotherapeutic agents.

Figures 10 to 13 present an overview via a general classification schema using the preferred marker genes. Cut off values are derived after expression analysis using HG-U133a arrays and MAS5.0 software using global scaling and target intensity settings of TGT = 500. In case two or more genes are used for a classification step, the expression values of the individual genes might be combined by first normalising each individual value and then combining the normalised values into a single meta-value. For example, the ESR1 expression value might be divided by e.g. 5000, the PGR expression value by e.g. 150, and the normalised values are added to a combined ESR1-PGR score. The expression value of IGKC (214669_s_at) might be divided by e.g. 2338, the value of CCL5 might be divided by e.g. 399 and both normalised values might then be added and subsequently divided by two in order to obtain an immune system metagen score. In addition, the ratio between stromal and proliferation might be deduced from normalised expression values of FBN1 and UBE2C by e.g. dividing the FBN1 value by e.g. 3366 and dividing the value of UBE2C by 973 and subsequently calculating a ratio between the normalised values. The constant used for normalisation are typically derived as the median expression value of that gene found in samples collected from a representative patient cohort. A representative patient cohort is a population of breast cancer patient of a) sufficient size, e.g. preferentially more than 30 breast cancer patients and b) preferentially containing a proportion of grade 1, 2, 3, as well as estrogen receptor positive and estrogen receptor negative tumors of at least 5 %.

However, the TAC study and the T-FAC study differ too much compared to the *in vitro* study. Therefore, the Paclitaxel prediction markers should be analyzed in further studies.

The results of the validations demonstrate that, although the validation studies included complex agent combinations and the effect of the other agents could not be analyzed, the predictive gene signatures for the single agents can classify sensitive and resistant tumors in a neoadjuvant combination therapy as well.

The question, which meaning the identified signatures might have for the clinical practice, has to be analyzed first in larger studies.

### Example 2

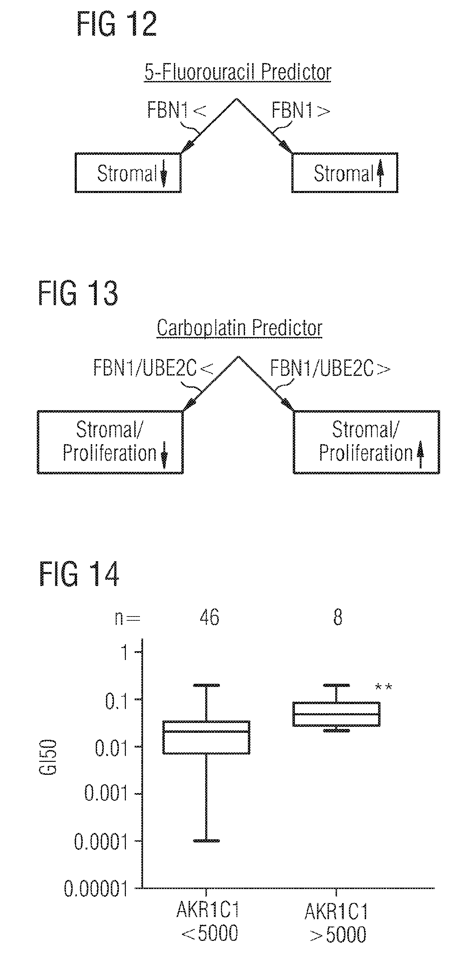

To prove the effect of the expression of AKR1C1 in cell lines, the study NCI 60 (Staunton et al., 2001) ²² was used. In this study several tumor cell lines of different cancer types were tested for their sensitivity towards several chemotherapeutic agents. The GI-50 value (concentration, where half of the tumor cells are inhibited regarding growing) for Paclitaxel was used to test, whether AKR1C1 is appropriate for a prediction of a Paclitaxel sensitivity in cell lines. The analysis demonstrates that in case of a high expression of AKR1C1 the cell lines mainly are resistant towards the agent Paclitaxel. The defined cut-off score (5000) allowed a significant classification (p=0.0049) of the resistant and sensitive tumor cells of the NCI 60 cell lines (Fig. 14).

In primary breast cancer tissues it was analyzed, which effect AKR1C1 has as predictive gene marker for Paclitaxel in the different subtypes defined in the *in vitro* study. Since in the *in vitro* study not enough tumor samples exist in the single subtypes to enable an appropriate analysis, the largest available validation dataset was used. The tumors of the neoadjuvant T-FAC study (Hess et al., 2006) ¹⁸ have been classified via the defined predictive gene signatures for Paclitaxel into four subtypes. Subsequently, it could be analyzed to what extent the AKR1C1 gene expression in the subtypes differs in the patients, who received a complete remission due to the neoadjuvant therapy, compared to the rest.

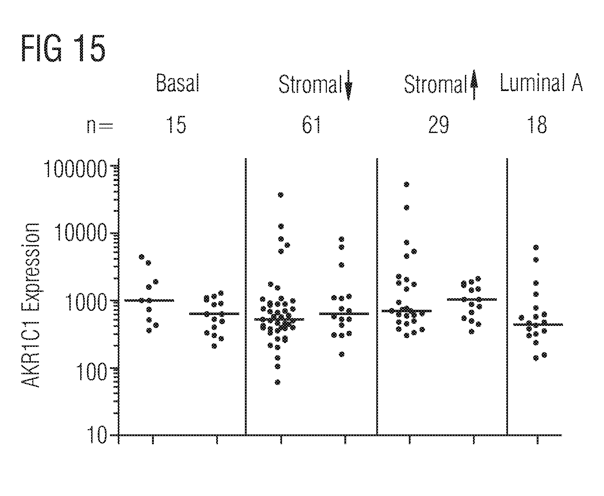

Within the basal subtype tumors with higher expression of AKR1C tended to receive an incomplete remission (trend for significance p=0.09; Fig. 15).

### Discussion

Only for the basal subtype it was possible regarding the expression of AKR1C1 to differ between resistant and sensitive tumors with a significant trend. This observation should be verified in subsequent studies.

In case it is possible to identify with this gene for the basal subtype tumors, which do not respond to Paclitaxel, this would be very important for a therapy. Patients, whose tumors belong to the basal subtype, have very often a poor clinical prediction since this tumor entity is very aggressive. At the same time, this tumor subtype responds very strong towards chemotherapeutic agents. Therefore, especially for this subtype it is important to elucidate resistance mechanisms to improve the life span via an optimal treatment.

### Figures

Figures 1 - 4 demonstrate the chemosensitivity of the tumors towards the chemotherapeutic agents Epirubicin (Fig. 1), Paclitaxel (Fig. 2), 5-Fluorouracil (Fig. 3) and Carboplatin (Fig. 4) after classification with predictive gene signatures. The results are depicted via a Box-Whisker-Plot of the AUC (Area under the dose-response curve) of the prediction classes for Epirubicin (basal, immune system-high, immune system-low, luminal A), Paclitaxel (basal, stromal-low, stromal-high, luminal A), 5-Fluorouracil (stromal-low, stromal-high) and Carboplatin (ratio stromal/proliferation-low, ratio stromal/proliferation-high) (43 tumors for the agents Paclitaxel/Epirubicin and 34 tumors for 5-Fluorouracil/Carboplatin). Significant results of a nonparametric test are demonstrated for the comparison of all subtypes adjacent the Figures and for the pairwise comparison of the subtypes within the Figures (* : p-score < 0.05, **: p-score < 0.01 , ***: p-score < 0.001) (Tab. 9).

Figure 5 demonstrates the relative reduction of 86 tumors towards a neoadjuvant EC combination therapy. The results are depicted via a Box-Whisker-Plot of the relative tumor reduction of the Epirubicin prediction classes (basal, immune system-high, immune system-low and luminal A). Significant results of a nonparametric test are demonstrated for the comparison of all subtypes adjacent the Figure and for the pairwise comparison of the subtypes within the Figure (* : p-score < 0.05, **: p-score < 0.01, ***: p-score < 0.001) (Tab. 9).

Figures 6 and 7 demonstrate the relative reduction of 39 tumors towards a neoadjuvant FEC combination therapy. The results are depicted via a Box-Whisker-Plot of the relative tumor reduction of the prediction classes, which are defined via the Epirubicin (Fig. 6) and 5-Fluorouracil (Fig. 7) prediction markers. Significant results of a nonparametric test are demonstrated for the comparison of all subtypes adjacent the Figures and for the pairwise comparison of the subtypes within the Figures (* : p-score < 0.05) (Tab. 9).

Figures 8 and 9 demonstrate prediction classes of the Paclitaxel prediction markers in neoadjuvant studies. The percentage of the tumors with "(pathological) complete remission" (pCR/CR) (in each case the bar on the left side) and with incomplete remission (in each case the bar on the right side) in a neoadjuvant TAC (Fig. 8) and T-FAC (Fig. 9) study is demonstrated.

Figures 10 to 13 demonstrate the general classification schema using preferred marker genes for the different chemotherapeutic agents.

Figure 14 demonstrates the chemosensitivity of different cell lines towards Paclitaxel. NCI 60 cell lines were classified with the marker gene AKR1C1 (cut-off score 5000). Onto the ordinate the concentration of Palitaxel is depicted, which is needed for the inhibition of half of the cells (GI-50). Altogether for Paclitaxel 54 GI-50 values for the cell lines were provided.

Figure 15 demonstrates the expression of the gene AKR1C1 in a neoadjuvant T-FAC study for the molecular subtypes basal, stromal-low, stromal-high and luminal A. For each group there occurred a classification of the tumors, which received a pathological complete remission (depicted in each case on the right side) or an incomplete remission (depicted in each case on the left side) towards the neoadjuvant therapy.

The figures are described in the context of the respective examples.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

**Table 7: Genes of Interest**

| Affymetrix Probeset ID | Gene symbol | Gene name |
|---|---|---|
| 218211_s_at | MLPH | melanophilin |
| 205225 at | ESR1 | estrogen receptor 1 |
| 208305 at | PGR | progesterone receptor |
| 205713_s_at | COMP | cartilage oligomeric matrix protein |
| 214669_x_at | IGKC | Immunoglobulin kappa variable 1-5 |
| 1405_i_at | CCL5 | chemokine (C-C motif) ligand 5 |
| 209335 at | DCN | decorin |
| 202766 s at | FBN1 | fibrillin 1 (Marfan syndrome) |
| 202954_at | UBE2C | ubiquitin-conjugating enzyme E2C |
| 204151_x_at | AKR1C1 | aldo-keto reductase family 1, member C1 (dihydrodiol dehydrogenase 1) (20-alpha (3-alpha)hydroxysteroid dehydrogenase) |

**Table 8: Primer sequences and probe sequences used in accordance with the present invention**

| Gene symbol | Oligonucleotide | Sequence | SEQ ID |
|---|---|---|---|
| | | | |
| MLPH | Probe | CCAAATGCAGACCCTTCAAGTGAGGC | 1 |
| | for-Primer | TCGAGTGGCTGGGAAACTTG | 2 |
| | rev-Primer | AGATAGGGCACAGCCATTGC | 3 |
| | | | |
| ESR1 | Probe | ATGCCCTTTTGCCGATGCA | 4 |
| | for-Primer | GCCAAATTGTGTTTGATGGATTAA | 5 |
| | rev-Primer | GACAAAACCGAGTCACATCAGTAATAG | 6 |
| | | | |
| PGR | Probe | TTGATAGAAACGCTGTGAGCTCGA | 7 |
| | for-Primer | AGCTCATCAAGGCAATTGGTTT | 8 |
| | rev-Primer | ACAAGATCATGCAAGTTATCAAGAAGTT | 9 |
| | | | |
| COMP | Probe | CAGGAGAACATCATCTGGGCCAACCTG | 10 |
| | for-Primer | GACAGCAACGTGGTCTTGGA | 11 |
| | rev-Primer | ATGGTGTCATTGCAGCGGTAA | 12 |
| | | | |
| IGKC | Probe | AGCAGCCTGCAGCCTGAAGATTTTGC | 13 |
| | for-Primer | GATCTGGGACAGAATTCACTCTCA | 14 |
| | rev-Primer | GCCGAACGTCCAAGGGTAA | 15 |
| | | | |
| CCL5 | Probe | CTCGGACACCACACCCTGCTGCT | 16 |
| | for-Primer | CTGCATCTGCCTCCCCATA | 17 |
| | rev-Primer | AGTGGGCGGGCAATGTAG | 18 |
| | | | |
| DCN | Probe | TCTTTTCAGCAACCCGGTCCA | 19 |
| | for-Primer | AAGGCTTCTTATTCGGGTGTGA | 20 |
| | rev-Primer | TGGATGGCTGTATCTCCCAGTA | 21 |
| | | | |
| FBN1 | Probe | CTCAGTGGCCAGAGGATCACCAGTGC | 22 |
| | for-Primer | GTCTGGGAGGACCAGGAAACA | 23 |
| | rev-Primer | TGCACATGCTGTGATGAAGGA | 24 |
| | | | |
| UBE2C | Probe | TGAACACACATGCTGCCGAGCTCTG | 25 |
| | for-Primer | CTTCTAGGAGAACCCAACATTGATAGT | 26 |
| | rev-Primer | GTTTCTTGCAGGTACTTCTTAAAAGCT | 27 |
| | | | |
| AKR1C1 | Probe | CCTATGCGCCTGCAGAGGTTCCTAAAAG | 28 |
| | for-Primer | CATGCCTGTCCTGGGATTTG | 29 |
| | rev-Primer | AATTTGGTGGCCTCTAAAGCTTT | 30 |

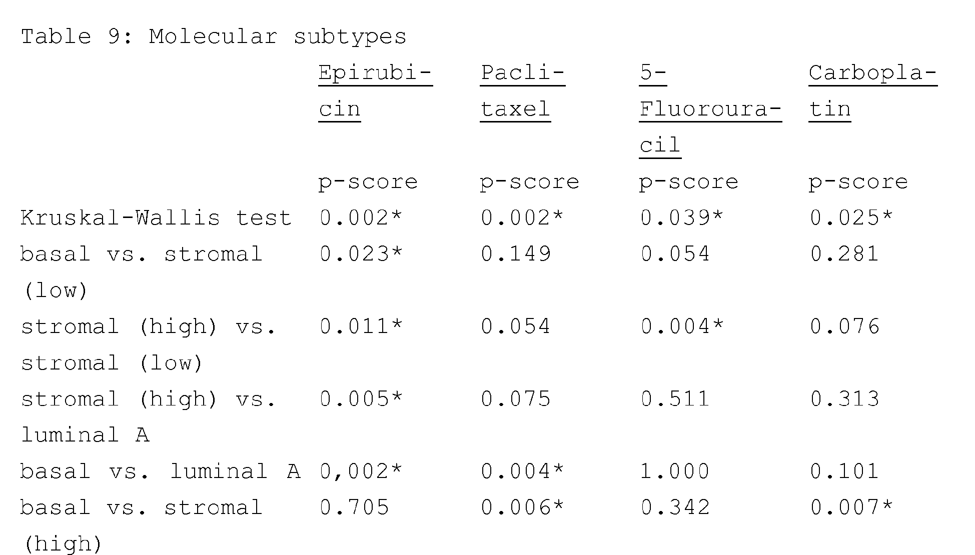

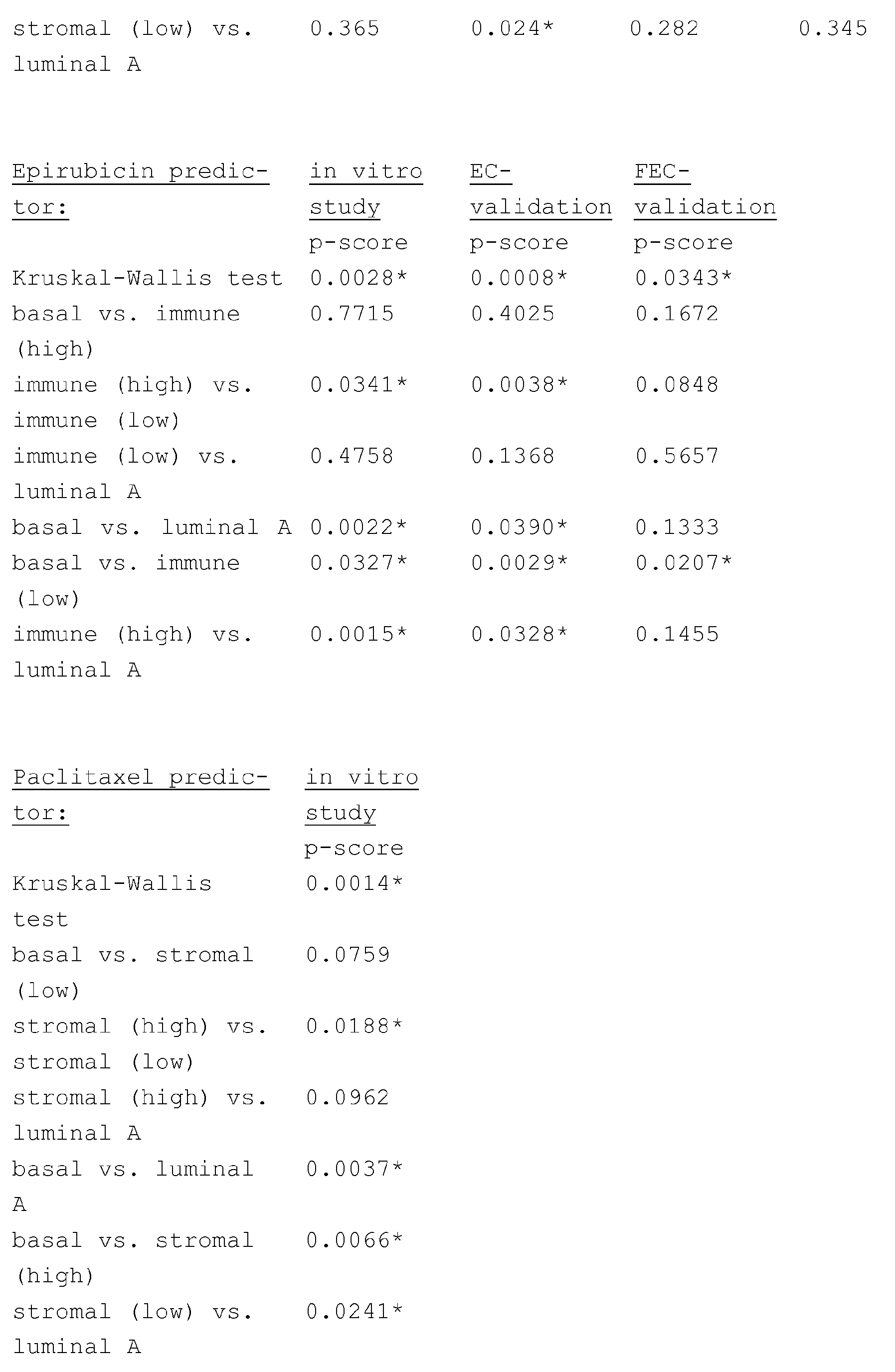

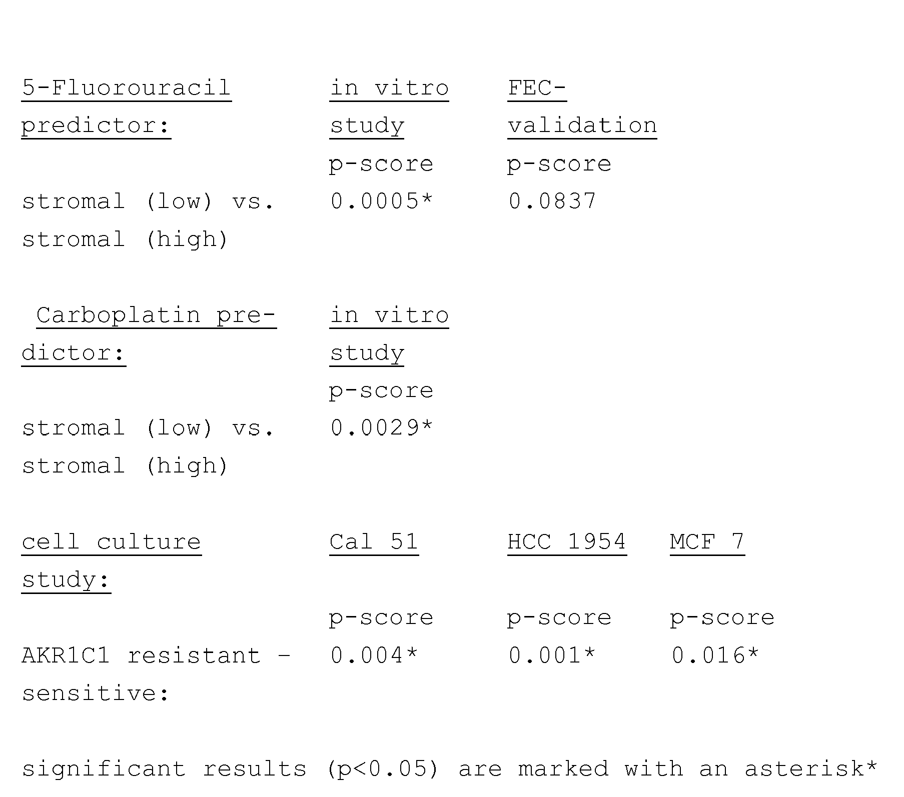

### References

1. Jemal A et al., CA Cancer J Clin 2007; 57:43-66.
2. Veronesi U et al., Lancet 2005; 365:1727-1741.
3. Gesellschaft der epidemiologischen Krebsregister in Deutschland e.V. (GEKID) und RKI, Krebs in Deutschland 2006; 5.Auflage, Saarbrücken 2006.
4. Bast RC JR et al., J Clin Oncol 2001; 19: 1865-1878.
5. Goldhirsch A et al., Ann Oncol 2005; 16: 1569-83.
6. Longley DB et al., Nat Rev Cancer 2003; 3: 330-338.
7. Capranico G et al., Cancer Res 1989; 49: 2022-2027.
8. Misset JL et al., J Clin Oncol 1996; 14: 1136-1145.
9. Nabholtz JM et al., J Clin Oncol 1999; 17: 1413-1424.
10. Henderson IC et al., J Clin Oncol 2003; 21: 976-83.
11. Bishop JF et al., J Clin Oncol 1999; 17: 2355-64.
12. Parness J and Horwitz SB J Cell Biol 1981; 191: 479-87.
13. Paridaens R et al., J Clin Oncol 2000; 18: 724-733.
14. Villeneuve DJ et al., Breast Cancer Res Treat 2006; 96:17-39.
15. Gyorffy B et al., Int J Cancer 2006; 118:1699-712.
16. Ross DT and Perou CM, Dis Markers 2001; 17:99-109.
17. Ayers M et al., J Clin Oncol 2004; 22:2284-2293.
18. Hess KR et al., J Clin Oncol 2006; 24:4236-44.
19. Gianni L et al., J Clin Oncol 2005; 23:7265-77.
20. Thuerigen O et al., J Clin Oncol 2006; 24:1839-45.
21. Rody A et al., Zentralbl Gynakol 2006; 128: 76-81.
22. Staunton JE et al., Proc Natl Acad Sci USA 2001; 98: 10787-92.

## Claims

1. A method for predicting a response of a tumor in a patient suffering from or at risk of developing recurrent gynecologic cancer towards a chemotherapeutic agent, said method comprising the steps of:
a) obtaining a biological sample from said patient;
b) determining the pattern of expression level of at least one gene of the group comprising AKR1C1, MLPH, ESR1, PGR, COMP, DCN, IGKC, CCL5, FBN1 and/or UBE2C, or of genes coregulated therewith, in said sample;
c) comparing the pattern of expression levels determined in (b) with one or several reference pattern(s) of expression levels;
d) identifying at least one marker gene;
e) determining a molecular subtype for said sample on the basis of (d); and
f)predicting from said molecular subtype response of a tumor for a chemotherapeutic agent,
wherein the molecular subtype is selected from the group comprising the subtypes basal, stromal-high, stromal-low, luminal A, immune system-high, immune system-low, proliferation-high and/or proliferation-low.

2. The method according to claim 1, said method comprising the further step of:
g) selecting at least one chemotherapeutic agent which is predicted to be successful,
wherein the chemotherapeutic agent is selected from the group comprising Epirubicin, Paclitaxel, 5-Fluorouracil and/or Carboplatin.

3. The method according to any one of the preceding claims, wherein
a) the basal molecular subtype predictive for Epirubicin sensitivity is **characterized by** down-regulated MLPH-expression, the luminal A molecular subtype predictive for Epirubicin resistance is **characterized by** up-regulated ESR1 and PGR expression, the immune system-high molecular subtype predictive for Epirubicin sensitivity is **characterized by** up-regulated IGKC and CCL5 expression, and/or the immune system-low molecular subtype predictive for Epirubicin resistance is **characterized by** down-regulated IGKC and CCL5 expression;
b) the basal molecular subtype predictive for Paclitaxel sensitivity is **characterized by** down-regulated MLPH expression, the luminal A molecular subtype predictive for Paclitaxel resistance is **characterized by** up-regulated ESR1 and PGR expression, the stromal-low molecular subtype predictive for Paclitaxel sensitivity is **characterized by** down-regulated DCN expression, and/or the stromal-high molecular subtype predictive for Paclitaxel resistance is **characterized by** up-regulated DCN expression;
c) the stromal-low molecular subtype predictive for 5-Fluorouracil sensitivity is **characterized by** down-regulated FBN1 expression, and/or the stromal-high molecular subtype predictive for 5-Fluorouracil resistance is **characterized by** up-regulated FBN1 expression; and/or
d) the stromal/proliferation-low molecular subtype predictive for Carboplatin sensitivity is **characterized by** down-regulated FBN1/UBE2C expression ratio, and/or the stromal/proliferation-high molecular subtype predictive for Carboplatin resistance is **characterized by** up-regulated FBN1/UBE2C expression ratio.

4. The method according to any one of the preceding claims, wherein Paclitaxel resistance in the basal molecular subtype is **characterized by** up-regulated AKR1C1 expression.

5. The method according to any one of the preceding claims, wherein the expression level is determined by
a) a hybridization based method;
b) a PCR based method;
c) determining the protein level, and/or by
d) an array based method.

6. The method to any one of the preceding claims, **characterized in that** the expression level of at least one of the said genes is determined with rtPCR (reverse transcriptase polymerase chain reaction) of the gene related mRNA.

7. The method according to any one of the preceding claims, **characterized in that** the expression level of at least one of the said genes is determined in formalin and/or paraffin fixed tissue samples.

8. The method according to any one of the preceding claims, wherein, after lysis, the samples are treated with silica-coated magnetic particles and a chaotropic salt, in order to purify the nucleic acids contained in said sample for further determination.

9. The method according to any one of the aforementioned claims **characterized in that** said gynecologic cancer is selected from the group comprising breast cancer, ovarian cancer, vulvar cancer, vaginal cancer, tubal cancer, endometrian cancer and/or cervical cancer.

10. The method according to any one of the aforementioned claims **characterized in that** said gynecologic cancer is breast cancer.

11. A kit useful for carrying out a method of any one of the preceding claims, comprising at least
a) a primer pair and/or a probe each having a sequence sufficiently complementary to a marker gene according to any one of the preceding claims; and/or
b) at least an antibody directed against a marker according to any one of the preceding claims.

12. A method for correlating the clinical outcome of a patient suffering from or at risk of developing recurrent gynecologic cancer with the presence or non-presence of a defect in marker gene expression, said method comprising the steps of:
a. obtaining a biological sample from said patient;
b. determining the expression level of at least one marker gene according to any one of the preceding claims in said patient, and
c. correlating the pattern of expression levels determined in (b) with said patient's data, said data being selected from the group consisting of etiopathology data, clinical symptoms, anamnesis data and/or data concerning the therapeutic regimen.

13. A nucleic acid molecule, selected from the group comprising
a) the nucleic acid molecule presented as SEQ ID NO: 1 - 30,
b) a nucleic acid molecule having a length of 4 - 80 nucleotides, preferably 18 - 30 nucleotides, the sequence of which corresponds to the sequence of a single stranded fragment of a gene encoding for a marker from the group comprising MLPH, ESR1, PGR, COMP, DCN, IGKC, CCL5, FBN1, UBE2C and/or AKR1C1,
c) a nucleic acid molecule that is a fraction, variant, homologue, derivative, or fragment of the nucleic acid molecule presented as SEQ ID NO: 1 - 30,
d) a nucleic acid molecule that is capable of hybridizing to any of the nucleic acid molecules of a) - c) under stringent conditions,
e) a nucleic acid molecule that is capable of hybridizing to the complement of any of the nucleic acid molecules of a) - d) under stringent conditions,
f) a nucleic acid molecule that is capable of hybridizing to the complement of a nucleic acid molecule of e),
g) a nucleic acid molecule having a sequence identity of at least 95 % with any of the nucleic acid molecules of a) - f),
h) a nucleic acid molecule having a sequence identity of at least 70 % with any of the nucleic acid molecules of a) - f),
i) a complement of any of the nucleic acid molecules of a) - h), and/or
j) a nucleic acid molecule that comprises any nucleic acid molecule of a) - i).

14. The nucleic acid according to claim 13, **characterized in that** the said nucleic acid is selected from the group comprising DNA, RNA, PNA, LNA and/or Morpholino.

15. The nucleic acid according to any of claims 13 - 14, **characterized in that** it is labelled with at least one detectable marker.

16. A kit of primers and/or detection probes, comprising at least one of the nucleic acids according to any of claims 13 - 15 and/or their fractions, variants, homologues, derivatives, fragments, complements, hybridizing counterparts, or molecules sharing a sequence identity of at least 70%, preferably 95 %.

17. The kit according to claim 16, comprising at least one of the nucleic acid molecules presented as SEQ ID NO: 1 - 30 and/or their fractions, variants, homologues, derivatives, fragments, complements, hybridizing counterparts, or molecules sharing a sequence identity of at least 70%, preferably 95 %, for the detection of at least one marker gene according to any one of the preceding claims.

18. Use of a nucleic acid according to any of claims 13 - 15 or of a kit according to any of claims 16 - 17 for predicting a clinical response of a patient suffering from or at risk of developing recurrent gynecologic cancer towards a chemotherapeutic agent.
